# EUROPEAN PATENT APPLICATION

(11) **EP 3 868 244 A1**
(43) Date of publication of application: **25.08.2021**
(21) Application number: 19874516.8
(22) Date of filing: 12.09.2019
(51) Int. Cl.: A43B 17/00, A61H 39/04

(54) **WARM PATCH FOR FEET**

(30) Priority: 17.10.2018 WO PCT/CN2018/110643
(71) Applicant: Kao Corporation, Chuo-ku, Tokyo 103-8210 (JP)
(72) Inventor: XING, Guangyun, Tokyo 131-8501 (JP); YAMAZAKI, Ritsuko, Shanghai 200241 (CN)
(74) Representative: Hoffmann Eitle
(86) International application number: PCT/CN2019/105622
(87) International publication number: WO 2020/078155

(57) **Abstract**

Provided is a warm patch (100) for feet, wherein same has protruding portions (12) and a warming portion. The warm patch comprises multiple protruding portions (12), and a substrate sheet (10) provided with the multiple protruding portions (12), wherein the substrate sheet (10) is made of a material including a non-woven fabric; the warming portion has a heating material; and the protruding portion (12) is made of a material including a non-woven fabric and is configured at a surface of the warm patch (100) for feet.

## Description

### Technical field

The invention relates to a warming patch for foot and a using method thereof.

### Background

In Patent Document 1, a warming patch for foot (a disposable warming patch for foot in this document) is described as a foot warming patch for stimulating acupuncture points on a foot sole. The warming patch for foot in Patent Document 1 is provided with magnets as protrusion portions for stimulating acupuncture points, which are formed in a flat semi-elliptical shape and the like.

Patent Document 1: Japanese Laid-Open Patent Publication No. 2006-26370

### Summary of Invention

The invention provides a warming patch for foot having a protrusion portion and a warming portion, wherein:
there are:
a plurality of the protrusion portions; and
a base material sheet provided with the plurality of the protrusion portions,
the base material sheet is constituted by a material including nonwoven fabrics,
the warming portion has a heat-generating material,
the protrusion portion is constituted by a material including nonwoven fabrics and is disposed on a surface of the warming patch for foot.

### Brief descriptions of the drawings

Fig. 1 is a top view of a warming patch for foot according to a first embodiment of the invention.
Figure 2 is a cross-sectional view of the warming patch for foot taken along line A-A of Figure 1.
Fig. 3 is a bottom view of the warming patch for foot according to the first embodiment.
Fig. 4 is a schematic diagram for explaining the arrangement of the protrusion portion in the warming patch for foot according to the first embodiment.
Fig. 5 is a schematic side view for explaining a modified example of the protrusion portion in the warming patch for foot.
Fig. 6 is a top view for explaining the arrangement of the protrusion portion in the warming patch for foot according to a second embodiment.
Fig. 7 is a perspective view of a use example of the warming patch for foot according to the second embodiment.
Fig. 8 is a perspective view of a second sheet of the warming patch for foot according to a third embodiment.
Fig. 9 is a top view of the second sheet of the warming patch for foot according to the third embodiment.
Fig. 10 is a cross-sectional view of the second sheet taken along line A-A of Figure 9.
Fig. 11 is a top view of the first sheet of the warming patch for foot according to the third embodiment.
Fig. 12 is a perspective view illustrating a use example of the warming patch for foot according to the third embodiment.
Fig. 13 is a cross-sectional view of the second sheet of the warming patch for foot according to a fourth embodiment.
Fig. 14 is a cross-sectional view of the protrusion portion and the nearby of the warming patch for foot according to a fifth embodiment.
Fig. 15 is a top view of a warming patch for foot according to a sixth embodiment.

### Description of Embodiments

In the warming patch for foot of Patent Document 1, heat is transferred to skin by the warming patch portion other than the magnet. The protrusion portion made of magnet does not transfer heat, besides it creates a gap between the warming patch and skin. The air present in the gap hinders the heat transfer, thereby making it difficult for the heat of the warming patch to be sufficiently transferred to skin. Therefore, there is further possibility of improvement in the heat transfer effect of the protrusion portion.

The invention relates to a warming patch for foot, which has a structure excellent in heat transfer effect of a protrusion portion.

Hereinafter, a preferable embodiment of the invention will be described with reference to the accompanying drawings. In addition, in all the drawings, like reference numerals refer to like constitute elements, and repeated descriptions are omitted as appropriate.

The invention relates to a warming patch for foot having a protrusion portion and a warming portion, which is provided with a plurality of protrusion portions and a base material sheet provided with the above-mentioned plurality of protrusion portions, the above-mentioned base material sheet is constituted by a material including nonwoven fabrics, the above-mentioned warming portion has a heat-generating material, and the above-mentioned protrusion portion is constituted by a material including the nonwoven fabric and are disposed on a surface of the warming patch for foot. The invention has various embodiments as described below.

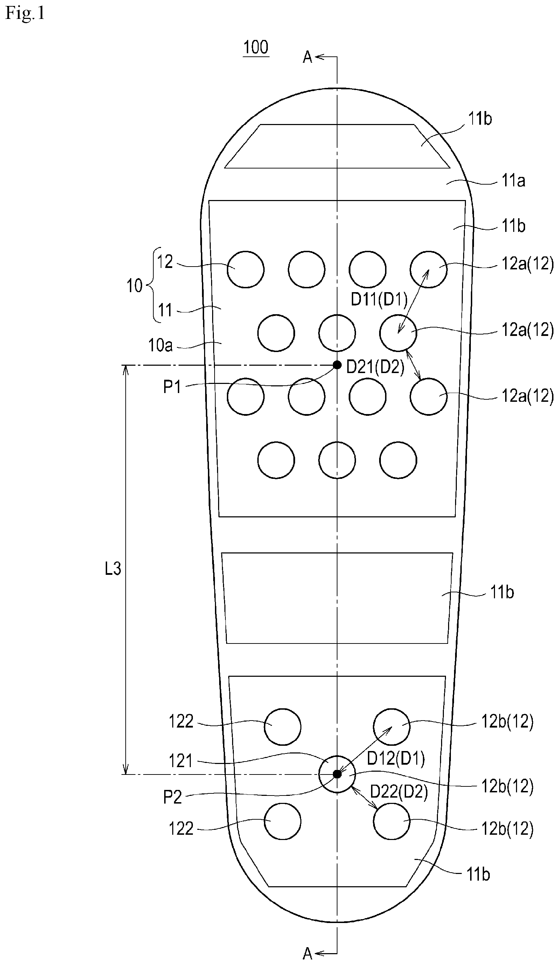

First, description will be made taken the first embodiment as an example and with reference to Figs.1 to 5. The description is basically applicable to other embodiments as well and will be described each time when different.

### (First embodiment)

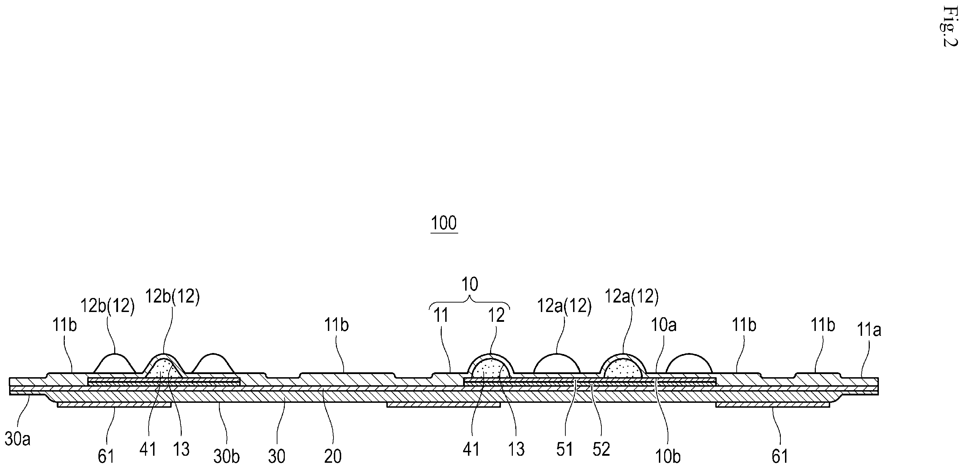

As shown in Fig. 1 or Fig. 2, the warming patch for foot 100 of the present embodiment is a warming patch for foot 100 having a protrusion portion 12 and a warming portion and is provided with a plurality of protrusion portions 12 and a base material sheet (for example, a first sheet 10) provided with the plurality of the protrusion portions 12.

In the invention, the protrusion portion 12 can be formed separately with respect to the first sheet 10 and fixed to the first sheet 10 by adhesion and the like, or it can be formed integrally with the first sheet 10 (formed as a part of the first sheet 10). In the present embodiment, the protrusion portion 12 is formed as a part of the first sheet 10.

The first sheet 10 is constituted by a material including nonwoven fabrics.

The warming portion has a heat-generating material 41. In the case of the present embodiment, the warming portion is configured by filling the cavity 13 inside the protrusion portion12 with the heat-generating material 41.

The protrusion portion 12 is constituted by a material including nonwoven fabrics and is disposed on a surface of the warming patch for foot 100.

According to the warming patch for foot 100 of the present embodiment, the warming patch for foot 100 is used in a state in which the protrusion portion 12 is closely pressed against skin of a sole of a human body, and thus, skin of the foot sole is pressed by the protrusion portion 12, while the heat emitted from the warming portion is more sufficiently transferred to skin of the foot sole via the protrusion portion 12, and is uniformly diffused to skin of the foot sole over a large area, thus enabling skin to be heated over a large area. As a result, the meridians and acupoints of the foot sole can be stimulated by pressing and warming, as in acupuncture. Therefore, it can be expected that the fatigue of the feet can be eliminated, and the sleep quality of the user can be improved.

The protrusion portion 12 is constituted by nonwoven fabrics, and since the nonwoven fabric has an appropriate elasticity and hardness, the nonwoven fabric is extended and gradually deformed under a pressure such as a weight, but it does not collapse completely. Therefore, in accordance with the load resistance control described in Paragraph 4 of Page 5 to be described later, the degree of pressing into the acupuncture points and skin of the foot sole is gradually reduced, and there is no excessive pain and irritation even when the warming patch is used for a long time, and the acupuncture points and skin of the foot sole can be massaged with an appropriate degree.

In addition, the warming patch for foot 100 is hermetically contained in a packaging material (not shown) in a state before use. When the packaging material is opened and the warming patch for foot 100 is taken out of the packaging material, oxygen contained in the outside air is supplied to the heat-generating material 41, thereby causing the heat-generating material 41 to generate heat.

There is no particular limit to the shape of the protrusion portion 12, for example, it can be a shape tapering toward the top end. However, it is preferable that the top end of the protrusion portion 12 is designed as a nearly circular shape.

The shape of the protrusion portion12 can be designed as, for example: a taper shape such as a conical shape, an elliptical conical shape, a long conical shape; or a frustum shape such as a truncated cone shape, an elliptical truncated cone shape, or a long-truncated cone shape; or a hemispherical shape; and the like.

The heat generating performance of the heat-generating material 41 is, for example, preferably set such that the temperature of the surface of skin becomes from 37°C to 44°C and more preferably from 38°C to 42°C.

When the protrusion portion 12 is pressed in a direction perpendicular to the plane of the first sheet 10 in a state of the first sheet 10 that the cavity 13 of the protrusion portion 12 is not filled with the heat-generating material 41, the load resistance of the protrusion portion 12 is preferably set as follows: that is, for each protrusion portion 12, if a force of 1 N is applied, the protrusion portion 12 is actually not plastically deformed but deformed within the elastic deformation range, and if a force of 60 N is applied, the protrusion portion 12 is plastically deformed; the load resistance of the protrusion portion 12 is more preferably set as follows: that is, for each protrusion portion 12, if a force of 5 N is applied, the protrusion portion 12 is actually not plastically deformed but deformed within the elastic deformation range, and if a force of 40 N is applied, the protrusion portion 12 is plastically deformed.

By setting in this manner, it is possible to sufficiently press the sole skin of the foot with the protrusion portion 12, and it is possible to set the sinking degree of the protrusion portion 12 with respect to skin of the foot sole to an appropriate range.

Here, the direction perpendicular to the plane of the first sheet 10 refers to the normal direction of the first sheet 10 (the direction perpendicular to one surface 10a of the first sheet 10).

In addition, for the warming patch for foot 100 in which the cavity 13 of the protrusion portion 12 is filled with the heat-generating material 41, when the protrusion portion 12 is pressed in a direction perpendicular to the plane of the first sheet 10, it is preferable to set the load resistance of the portion of the warming patch for foot 100 corresponding to the protrusion portion 12 as follows: that is, for each protrusion portion 12, if a force of 3 N is applied, the protrusion portion 12 is actually not plastically deformed but deformed within the elastic deformation range; the load resistance of the portion of warming patch for foot 100 corresponding to the protrusion portion 12 is more preferably set as follows: that is, for each protrusion portion 12, if a force of 5 N is applied, the protrusion portion 12 is actually not plastically deformed but deformed within the elastic deformation range.

By setting in this manner, it is possible to sufficiently press the sole skin of the foot with the protrusion portion 12, and it is possible to set the sinking degree of the protrusion portion 12 with respect to skin of the foot sole to an appropriate range.

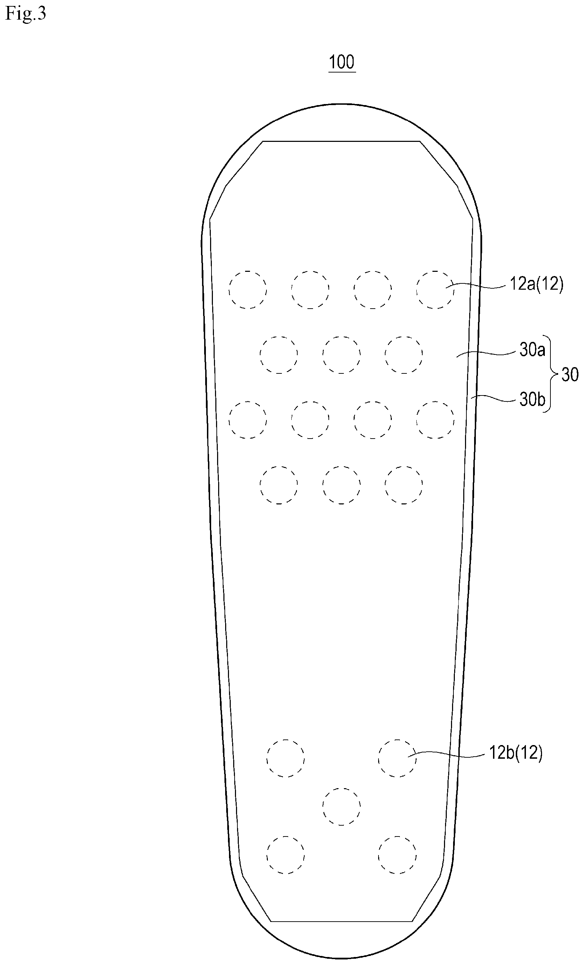

In the present embodiment, as illustrated in Figs. 1 and 3, the warming patch for foot 100 is designed to be in the shape of a human footprint. That is, the plan shape of the warming patch for foot 100 is a shape of a human footprint, and the warming patch for foot 100 is used in a state of being in close contact with a sole of a human foot.

In addition, in the following description, sometimes the tiptoe portion of the warming patch for foot 100 is referred as front (front side), and the heel portion is referred as rear (rear side).

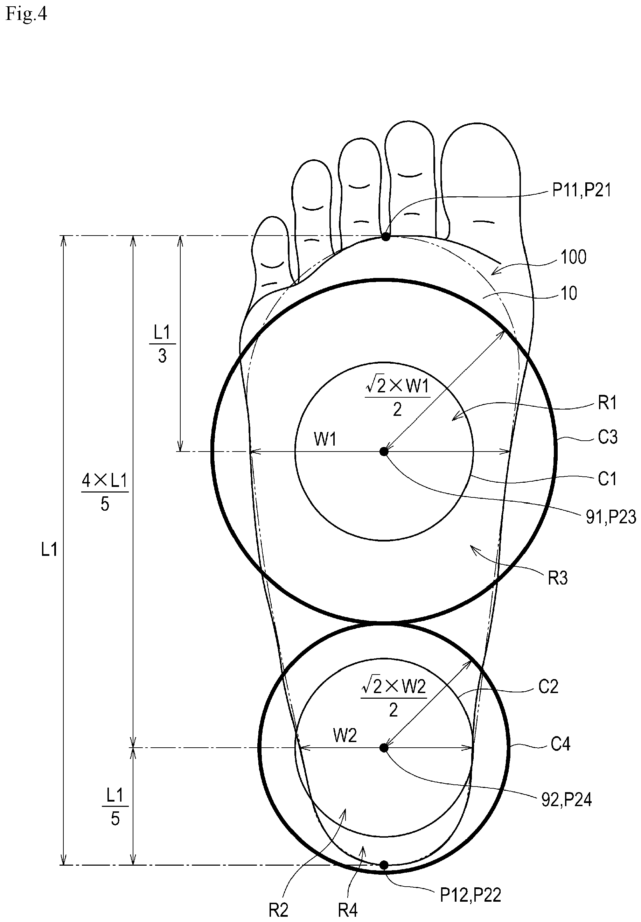

The plurality of protrusion portions 12 includes: a first protrusion portion 12a disposed at a position in the warming patch for foot 100 corresponding to the Yongquan acupuncture point 91 (see Fig. 4) on a sole of a human foot, and a second protrusion portion 12b disposed at a position in the warming patch for foot 100 corresponding to the insomnia acupuncture point 92 (see Fig. 4) on a sole of a human foot.

The disposing manner described above "disposed at a position corresponding to the Yongquan acupuncture point 91 on a sole of a human foot" is not limited to disposing any protrusion portion of the first protrusion portion 12a to directly contact the Yongquan acupuncture point 91. It also includes: Within a range that the effects of the invention can be obtained, the first protrusion portion 12a is disposed near the Yongquan acupuncture point 91, or the first protrusion portion 12a is disposed to surround the Yongquan acupuncture point 91.

Same as described above, the disposing manner "disposed at a position corresponding to the insomnia acupuncture point 92 on a sole of a human foot" is not limited to disposing any protrusion portion of the second protrusion portion 12b to directly contact the insomnia acupuncture point 92. It also includes: Within a range that the effects of the invention can be obtained, the second protrusion portion 12b is disposed near the insomnia acupuncture point 92, or the second protrusion portion 12b is disposed to surround the insomnia acupuncture point 92.

Accordingly, the Yongquan acupuncture point 91 and the insomnia acupuncture point 92 can be simultaneously pressed by using the warming patch for foot 100.

The shape of the human footprint is a shape along an outer shape of a sole of a human foot, and examples thereof may include a substantially oblong shape and a substantially elliptical shape as shown in Fig. 1. In more detail, for example, in the warming patch for foot 100, the width of the portion corresponding to the front portion of the foot is larger than the width of the portion corresponding to the rear portion of the foot. However, the warming patch for foot 100 may also be set such that: the width of the portion corresponding to the front portion of the foot and the width of the portion corresponding to the rear portion of the foot are equal to each other.

The warming patch for foot 100 may or may not have a portion for placing toes. In the case that the warming patch for foot 100 has a portion for placing toes, the shape of the footprint is a shape along the outer shape of the foot sole including the toes. In the case that the warming patch for foot 100 does not have a portion for placing toes, the shape of the footprint is a shape along the outer shape of the foot sole excluding the toes. In Fig. 4, the contour line of the warming patch for foot 100 is indicated by a double-dot line, and in the case of the present embodiment, the warming patch for foot 100 does not have a portion for placing toes.

Here, as shown in Fig. 4, the distance from the middle point P11 between the second toe root and the third toe root to the edge P12 of the heel is set to be L1. The Yongquan acupuncture point 91 is an acupuncture point located approximately L1/3 from the middle point P11 toward the heel. The insomnia acupuncture point 92 is an acupuncture point located approximately 4×L1/5 from the middle point P11 to the heel. In other words, the insomnia acupuncture point 92 is an acupuncture point located approximately L1/5 from the edge of the heel P12 toward the tiptoe.

The position corresponding to the Yongquan acupuncture point 91 is a position near the Yongquan acupuncture point 91 when the warming patch for foot 100 is tightly attached to the foot sole, and the position corresponding to the insomnia acupuncture point 92 is a position near the insomnia acupuncture point 92 when the warming patch for foot 100 is tightly attached to the foot sole.

There is no particular limit to the number of the first protrusion portion 12a, it may be one or more than one. As an example, the number of the first protrusion portions 12a may be 1 to 128.

There is no particular limit to the number of the second protrusion portions 12b, it may be one or more than one. As an example, the number of the second protrusion portions 12b may be 1 to 50.

As shown in Fig. 1, the distance L3 between the center position P1 of the arrangement region of the first protrusion portion 12a and the center position P2 of the arrangement region of the second protrusion portion 12b is preferably from 5 cm to 20 cm, and more preferably from 7 cm to 15 cm. "The center position P1 of the arrangement region of the first protrusion portion 12a" is the center position of a plurality of the first protrusion portion 12a when there is a plurality of the first protrusion portions 12a, and it is the center position of the first protrusion portion 12a when there is one first protrusion portion 12a. Similarly, "the center position P2 of the arrangement region of the second protrusion portion 12b" is the center position of a plurality of second protrusion portion 12b when there is a plurality of second protrusion portion 12b, and it is the center position of the second protrusion portion 12b when there is one second protrusion portion 12b.

There is no particular limit to the diameter of the protrusion portion 12, and it can be set for example, to be from 1 mm to 20 mm, preferably to be 15 mm or less.

There is no particular limit to the height of the protrusion portion 12, and it is preferably from 2 mm to 8 mm.

There is no particular limit to the height of the first protrusion portion 12a, and it is preferably from 4 mm to 8 mm.

There is no particular limit to the height of the second protrusion portion 12b, and it is preferably from 2 mm to 6 mm.

The height of the first protrusion portion 12a and the height of the second protrusion portion 12b may be the same or different from each other.

In addition, the insomnia acupuncture point 92 is located at the heel, therefore the sinking depth of the second protrusion portion 12b with respect to skin at the position of the insomnia acupuncture point 92 is suppressed to some extent, while the sinking depth of the first protrusion portion 12a with respect to skin at the position of the Yongquan acupuncture point 91 is easy to be deeper. Therefore, in the case that the height of the first protrusion portion 12a and the height of the second protrusion portion 12b are different from each other, the height of the first protrusion portion 12a is preferably higher than the height of the second protrusion portion 12b. However, the invention is not limited to this embodiment, and the height of the second protrusion portion 12b may also be higher than the height of the first protrusion portion 12a.

In addition, "the height of the first protrusion portion 12a is higher than the height of the second protrusion portion 12b" refers that the height of the first protrusion portion 12a is higher than either the height of the central protrusion portion 121 or the height of the peripheral protrusion portion 122.

More specifically, "the height of the first protrusion portion 12a is higher than the height of the second protrusion portion 12b " refers that the height of the first protrusion portion 12a may be higher than the height of at least any one of the peripheral protrusion portions, and the height of the first protrusion portion 12a may also be higher than the height of all the peripheral protrusion portions, and further, the height of the first protrusion portion 12a may be higher than the height of at least any one of the central protrusion portions 121, and the height of the first protrusion portion 12a may also be higher than the height of all the central protrusion portions 121.

In top view, there is no particular limit to the shortest distance D1 (Fig. 1) between vertexes of the adjacent protrusion portions 12, and it can be set to be 6 cm or less, preferably to be 0.5 cm or more.

In top view, there is no particular limit to the distance D11 (Fig. 1) between vertexes of the adjacent first protrusion portions 12a, and it can be set to be 6 cm or less, preferably to be 0.5 cm or more.

In top view, there is no particular limit to the distance D12 (Fig. 1) between vertexes of the adjacent second protrusion portions 12b, and it can be set to be 6 cm or less, preferably to be 0.5 cm or more.

In top view, there is no particular limit to the shortest distance (the shortest interval) D2 (Fig. 1) between the adjacent protrusion portion 12, and it can be set to be 4 cm or less, preferably to be 0.5 cm or more. The adjacent protrusion portions 12 may be connected to each other or may also be integrated with each other.

In top view, there is no particular limit to the distance (the interval) D21 (Fig. 1) between the adjacent first protrusion portions 12a, and it can be set to be 4 cm or less, preferably to be 0.5 cm or more. The adjacent first protrusion portions 12a may be connected to each other or may also be integrated with each other.

In top view, there is no particular limit to the distance (the interval) D22 (Fig. 1) between the adjacent second protrusion portions 12b, and it can be set to be 4 cm or less, preferably to be 0.5 cm or more. The adjacent second protrusion portions 12b may be connected to each other or may also be integrated with each other.

The distance D11 and the distance D12 may be equal to and different from each other and one distance may also be larger than the other.

In the present embodiment, the distance D11 and the distance D12 are set to be equal to each other.

However, the invention is not limited to this embodiment, and the distance D11 is preferably larger than the distance D12.

That is, as an example, the warming patch for foot 100 has a plurality of first protrusion portions 12a and a plurality of second protrusion portions 12b, and the distance D11 between vertexes of the first protrusion portions 12a is larger than the distance D12 between vertexes of the second protrusion portions 12b.

As shown in Fig. 4, in the warming patch for foot 100, the distance from the middle portion P21 corresponding to the middle (middle point P11) between the second toe root and the third toe root to the edge P22 of the heel of the warming patch for foot 100 is also set to be L1.

There is no particular limit to the length of the distance LI, and for example, it can be set to be from 13 cm to 25 cm.

In the present embodiment, the plurality of first protrusion portions 12a are disposed in: a first circular region R1 (a region within a circle C1 shown in Fig. 4), which is centered on the width center P23 of the warming patch for foot 100 located at L1/3 from the middle portion P21 toward the heel and has a radius of 3 cm. "The protrusion portion 12 is disposed in the first circular region R1" refers that at least a part of the protrusion portion 12 is in the first circular region R1.

In addition, in the present embodiment, the plurality of second protrusion portions 12b are disposed in: a second circular region R2 (a region within a circle C2 shown in Fig. 4), which is centered on the width center P24 of the warming patch for foot 100 located at 4×L1/5 from the middle portion P21 toward the heel and has a radius of 3 cm. "The protrusion portion 12 is disposed in the second circular region R2" refers that at least a part of the protrusion portion 12 is in the second circular region R2.

In this way, the warming patch for foot 100 is designed to be in a shape of a human footprint, and when the distance from the middle portion P21 corresponding to the middle of the second toe root and the third toe root to the edge P22 of the heel in the warming patch for foot 100 is set to be LI, the plurality of protrusion portions 12 includes: a first protrusion portion 12a disposed in the first circular region R1 which is centered on the width center P23 at L1/3 from the middle portion P21 toward the heel and has a radius of 3 cm; and a second protrusion portion 12b disposed in the second circular region R2 which is centered on the width center P24 at 4×L1/5 from the middle portion P21 toward the heel and has a radius of 3 cm.

In addition, the plurality of first protrusion portions 12a are disposed in: a range of ±3 cm in the longitudinal direction (front-rear direction) of the warming patch for foot 100, taken the position at L1/3 from the middle portion P21 toward the heel as a reference.

In addition, the plurality of second protrusion portions 12b are disposed in: a range of ±3 cm in the longitudinal direction (front-rear direction) of the warming patch for foot 100, taken the position at 4×L1/5 from the middle portion P21 toward the heel as a reference.

As shown in Fig. 4, the width of the warming patch for foot 100 at L1/3 from the middle portion P21 toward the heel is set to be W1. In addition, the width of the warming patch for foot 100 at 4×L1/5 from the middle portion P21 toward the heel is set to be W2.

There is no particular limit to the width W1, and for example, it can be set to be from 7 cm to 14 cm.

There is no particular limit to the width W2, and for example, it can be set to be from 4 cm to 11 cm.

In the present embodiment, the plurality of the first protrusion portions 12a is disposed in: a third circular region R3 (a region within a circle C3 shown in Fig. 4) having a center on the above-mentioned width center P23 and an area S1 of (W1)²×π/2. "The first protrusion portion 12a is disposed in the third circular region R3" refers that at least a part of the first protrusion portion 12a is located in the third circular region R3.

Further, in the present embodiment, the plurality of the second protrusion portions 12b is disposed in: a fourth circular region R4 (a region within a circle C4 shown in Fig. 4) having a center on the above-mentioned width center P24 and an area S2 of (W2)²×π/2. "The second protrusion portion 12b is disposed in the fourth circular region R4" refers that at least a part of the second protrusion portion 12b is located in the fourth circular region R4.

In this way, the warming patch for foot 100 is designed as the shape of a human footprint, and when the distance from the middle portion P21 corresponding to a middle of the second toe root and the third toe root to the edge P22 of the heel in the warming patch for foot 100 is set to be LI, the width of the warming patch for foot 100 at L1/3 from the middle portion P21 toward the heel is set to be W1, and the width of the warming patch for foot 100 at 4×L1/5 from the middle portion P21 toward the heel is set to be W2, the plurality of protrusion portions 12 include: a first protrusion portion 12a disposed in the third circular region R3 which is centered on the width center P23 at L1/3 from the middle portion P21 toward the heel and has an area S1 of (W1)²×π/2; and a second protrusion portion 12b disposed in the fourth circular region R4 which is centered on the width center P24 at 4×L1/5 from the middle portion P21 toward the heel and has an area S2 of (W2)²×π/2.

In the case of the present embodiment, in the warming patch for foot 100, a region that does not belong to either of the third circular region R3 or the fourth circular region R4 is a non-formed region in which the protrusion portion 12 is not formed.

Here, "the protrusion portion 12 is not formed" refers that the plane center of the protrusion portion 12 does not exist. That is, in the non-formed region of the warming patch for foot 100, there is no plane center of the protrusion portion 12.

Therefore, it is facilitated to intensively press the periphery of the Yongquan acupuncture point and the periphery of the insomnia acupuncture point.

In the case of the present embodiment, as shown in Fig. 1, the warming patch for foot 100 has a plurality of first protrusion portions 12a and a plurality of second protrusion portions 12b.

Besides, the configuration form of the plurality of first protrusion portions 12a and the configuration form of the plurality of second protrusion portions 12b are different from each other.

As an example, the plurality of the first protrusion portions 12a is configured to be in a shape of, for example, an alternate arrangement. More specifically, the warming patch for foot 100 is provided with the following in order from the tiptoe: a row in which four first protrusion portions 12a are arranged in the width direction, a row in which three first protrusion portions 12a are arranged in the width direction, a row in which four first protrusion portions 12a are arranged in the width direction, and a row in which three first protrusion portions 12a are arranged in the width direction. That is, the warming patch for foot 100 is provided with, for example, fourteen first protrusion portions 12a in total.

However, the configuration form of the plurality of first protrusion portions 12a in the invention is not limited to the example described herein.

Further, as an example, the plurality of second protrusion portions 12b includes: a central protrusion portion 121 disposed at a center of the plurality of the second protrusion portions 12b, and a plurality of peripheral protrusion portions 122 disposed on a circumference with the central protrusion portion 121 as a center.

In view of the stimulation to acupuncture points, it is preferable that: the size of the central protrusion portion 121 (at least any one of the height and the plane area) equals to or larger than the size of the peripheral protrusion portion 122.

On the other hand, in view that that the heat transfer from the plurality of the second protrusion portions 12b to skin is uniformly performed and the heat transfer to the center of the arrangement region of the plurality of the second protrusion portions 12b is appropriately suppressed, it is preferable that: the size (at least any one of the height and the plane area) of the central protrusion portion 121 is equal to or smaller than the size of the peripheral protrusion portion 122.

More specifically, the warming patch for foot 100 is provided with, for example, five second protrusion portions 12b in total, that is, one central protrusion portion 121 and four peripheral protrusion portions 122 arranged along a same circumference.

However, the configuration form of the plurality of second protrusion portions 12b in the invention is not limited to the example described herein.

Besides, in the invention, the configuration form of the plurality of the first protrusion portions 12a and the configuration form of the plurality of the second protrusion portions 12b can also be different from each other.

In the case of the present embodiment, as shown in Fig. 2, the shape of the top end of the second protrusion portion 12b is designed to be sharper than that of the top end portion of the first protrusion portion 12a.

More specially, for example, the first protrusion portion 12a is designed to have a hemispherical shape, and the second protrusion portion 12b is designed to have a conical shape.

However, in the invention, the shapes of the first protrusion portion 12a and the second protrusion portion 12b are not limited to the example described herein, and the shapes of the first protrusion portion 12a and the second protrusion portion 12b may also be same as each other.

In the case of the present embodiment, the protrusion portion 12 is curved into a convex shape on a surface of the warming patch for foot 100, and it is formed as a cavity 13 on the other surface.

More specifically, in the case of the present embodiment, the first sheet 10 is partially curved into a convex shape on the surface 10a of the first sheet 10, thereby forming the protrusion portion 12. The other surface 10b of the first sheet 10 is formed as a cavity 13 at the position where the protrusion portion 12 is formed.

The first sheet 10 is provided with: a flat sheet-like base portion 11, and a plurality of protrusion portions 12 curved into a convex shape on the surface 10a of the first sheet 10 with the base portion 11 as a reference.

The first sheet 10 forms an outer surface of the warming patch for foot 100. For example, the warming patch for foot 100 is used in a state in which the first sheet 10 (particularly, the protrusion portion 12) is in direct contact with skin of the foot sole.

In the case of the present embodiment, the first sheet 10 is constituted by a layer of nonwoven fabric sheet.

In the case of the present embodiment, the plane shape of the first sheet 10 is same as that of the warming patch for foot 100.

In addition, in the present embodiment, the cavity 13 of the protrusion portion 12 is filled with the heat-generating material 41. Therefore, the heat generated via the heat-generating material 41 is easily transferred through skin of the foot sole. In addition, the load resistance and the shape retention of the protrusion portion 12 are improved by filling the cavity 13 of the protrusion portion 12 with the heat-generating material 41.

The warming patch for foot 100 is provided with a second sheet 20, which is laminated on the other surface 10b of the first sheet 10. The second sheet 20 may be in a single-layer structure or a multi-layer structure.

In the case of the present embodiment, the plane shape of the second sheet 20 is same as that of the warming patch for foot 100. The first sheet 10 and the second sheet 20 are overlapped with each other and at least the peripheral portions are joined to each other in a state in which the contour lines of the first sheet 10 and the second sheet 20 coincide with each other. The heat-generating material 41 is held between the second sheet 20 and the first sheet 10.

That is, the warming patch for foot 100 is provided with the second sheet 20 which sandwiches the warming portion (heat-generating material 41) in the middle and is laminated on the other surface (the other surface 10b) of the base material sheet (the first sheet 10).

As shown in Figs. 1 and 2, in the present embodiment, the base portion 11 of the first sheet 10 has a first portion 11a with a relatively thin thickness and a second portion 11b with a relatively thick thickness. The first sheet 10 and the second sheet 20 are joined to each other at, for example, the first portion 11a by heat sealing.

As shown in Fig. 1, the first portion 11a is disposed at, for example: the peripheral section of the first sheet 10, the front part and the rear part of the arrangement region of the plurality of the first protrusion portions 12a, and the front part and the rear part of the arrangement region of the plurality of the second protrusion portions 12b.

The first portion 11a is further cured by heat sealing compared with the second portion 11b. Compared with the first portion 11a, the second portion 11b also has the texture of nonwoven fabric and has good skin tactility.

In the arrangement region of the protrusion portion 12, the warming patch for foot 100 is provided with a water absorption sheet laminated between the second sheet 20 and the base portion 11. As an example, the warming patch for foot 100 is provided with two water absorption sheets, that is, a first water absorption sheet 51 and a second water absorption sheet 52.

Since the warming patch for foot 100 is provided with the water absorption sheet, remaining water in the heat-generating material 41 can be absorbed by the water absorption sheet. Therefore, when the warming patch for foot 100 is taken out of the packaging material, the heat-generating material 41 can rapidly generate heat.

The water absorption sheet is disposed, for example, in the arrangement region of the plurality of the first protrusion portions 12a and the arrangement region of the plurality of the second protrusion portions 12b, respectively.

In addition, the warming patch for foot 100 may not include a water absorption sheet (not shown) depending on the composition of the heat-generating material 41 and the like, and the water absorption sheet may also be in a single-layer structure.

The warming patch for foot 100 is further provided with a third sheet 30 that sandwiches the second sheet 20 in the middle and is laminated on the other surface 10b of the first sheet 10. In the case of the present embodiment, the third sheet 30 constitutes the other outer surface of the warming patch for foot 100. In the case of the present embodiment, the plane shape of the third sheet 30 is same as that of the warming patch for foot 100.

The third sheet 30 is constituted by a nonwoven fabric sheet in a single-layer structure or a multi-layer structure.

The second sheet 20 and the third sheet 30 are overlapped with each other in a state where their contour lines coincide with each other.

In the case of the present embodiment, the second sheet 20 and the third sheet 30 are joined to each other at their peripheral sections.

As shown in Figs. 2 and 3, in the present embodiment, the third sheet 30 has a first portion 30a with a relatively thin thickness and a second portion 30b with a relatively thick thickness. The second sheet 20 and the third sheet 30 are joined to each other at, for example, the first portion 30a by heat sealing.

The first portion 30a is further cured by heat sealing compared with the second portion 30b. Compared with the first portion 30a, the second portion 30b also has the texture of nonwoven fabric and has good skin tactility.

In this way, the first portion 30a is disposed on, for example, the peripheral section of the third sheet 30, and the region of the third sheet 30 except the peripheral section becomes the second portion 30b.

In addition, for the thickness of the third sheet 30, the thickness in a region in which the third sheet 30 and the second sheet 20 are not joined is thicker than that in a region in which the third sheet 30 and the second sheet 20 are joined.

Here, the warming patch for foot 100 can be used in a state of being disposed along the upper surface of an insole (bottom) of a shoe such as a sandal or a slipper. Accordingly, an adhesive layer 61 (Fig. 2) for adhesively fixing the warming patch for foot 100 to the upper surface of the insole of a shoe may also be provided on the outer surface of the third sheet 30 (the surface of the third sheet 30 that is located opposite to the first sheet 10). In addition, an illustration of the adhesive layer 61 is omitted in Fig. 3.

Here, the arrangement region of the plurality of first protrusion portions 12a and the arrangement region of the plurality of second protrusion portions 12b overlap with the above-mentioned second portion 30b, respectively. Therefore, in the arrangement region of the plurality of first protrusion portions 12a and the arrangement region of the plurality of second protrusion portions 12b, the second sheet 20 and the third sheet 30 are in a non-joined state, and the second sheet 20 and the third sheet 30 can relatively move (slide and the like) in the surface direction thereof.

That is, the second sheet 20 and the third sheet 30 are partially joined to each other (for example, joined to each other at the peripheral section), and in the top view, the second sheet 20 and the third sheet 30 are not joined to each other in the arrangement region of the plurality of the first protrusion portions 12a and the arrangement region of the plurality of the second protrusion portions 12b.

Therefore, for example, when the warming patch for foot 100 is attached to the insole of a shoe and the like, the second sheet 20 and the third sheet 30 move relatively in the surface direction thereof when the user walks or changes directions during walking, thereby stress applied to the warming patch for foot 100 is absorbed, thereby a state in which the first protrusion portion 12a and the second protrusion portion 12b are stably attached to skin of the foot sole is kept, and positional deviation of the first protrusion portion 12a and the second protrusion portion12b from skin of the foot sole is suppressed. Further, since the first portion 30a has a large thickness and a high cushioning property, it is possible to appropriately cushion the pressing of the protrusion portion12 against skin.

The heat-generating material 41 includes, for example, an oxidizable metal, a water retaining agent, and water. The heat-generating material 41 generates heat by supplying oxygen to the oxidizable metal in the heat-generating material 41.

The heat-generating material 41 may also contain iron and carbon components.

The "iron" used herein may be at least a part of the above-mentioned oxidizable metal or may be a component different from the oxidizable metal, and typically, the "iron" is the above-mentioned oxidizable iron.

Here, the "carbon component" may be at least a part of the above-mentioned water retaining agent, and the heat-generating material 41 may also contain a carbon component except the water retaining agent.

In the warming patch for foot 100, the heat-generating material 41 may also be filled between the first sheet 10 and the second sheet 20 in a portion corresponding to the base portion 11, besides that the heat-generating material 41 is filled in the portion corresponding to the protrusion portion 12 of the first sheet 10. However, even in this case, it is preferable that: the thickness of the heat-generating material 41 in the portion corresponding to the protrusion portion 12 of the first sheet 10 is larger than that of the heat-generating material 41 in the portion corresponding to the base portion 11.

More preferable: the heat-generating material 41 is partially filled in the portion corresponding to the protrusion portion 12 of the first sheet 10, and there is no heat-generating material 41 in at least a part of the portion corresponding to the base portion 11.

In the case of the present embodiment, the heat-generating material 41 is filled in the portion of the first sheet 10 corresponding to the protrusion portion 12, while there is actually no heat-generating material 41 in the portion corresponding to the base portion 11.

It is preferable that the heat-generating material 41 is filled in the cavity 13 at a portion at least corresponding to the top end of the protrusion portion 12. Thus, skin can be heated by the top end of the protrusion portion 12.

For example, it is preferable that the heat-generating material 41 is filled in a region from the top end of the protrusion portion 12 to 50% or more in the height direction of the cavity 13.

More specifically, in the case of the present embodiment, the heat-generating material 41 is filled in, for example, a region of 70% or more in the height direction of the cavity 13.

More preferably, the heat-generating material 41 is filled in a region of 90% or more in the height direction of the cavity 13.

In the case of the present embodiment, for example, the other surface 10b of the base portion 11 of the first sheet 10 and the surface of the base end of the protrusion portion 12 of the heat-generating material 41 in the cavity 13 are flushed to each other.

Here, a method of measuring the filling ratio of the heat-generating material 41 in the height direction of the cavity 13 will be described.

A laser microscope or a laser displacement meter capable of measuring a height difference is used as the measuring device. A one-dimensional dot type, a two-dimensional laser displacement meter, a three-dimensional laser displacement meter, and the like can be used as the laser displacement meter. According to the shape and the height of the protrusion portion 12, an appropriate laser displacement meter is selected based on the distance required to be measured and the dot distance of the laser light. For example, a sensor IL-300 manufactured by Keyence Co., Ltd., (measurement distance: 160mm∼450mm, dot diameter ϕ: 500µm) can be used.

During the measurement, the third sheet 30, the second sheet 20, and the water absorption sheets (the first water absorption sheet 51 and the second water absorption sheet 52) are removed from the first sheet 10 in a nitrogen atmosphere in a manner that the heat-generating material 41 does not generate heat, so that the surface on the base end of the protrusion portion 12 of the heat-generating material 41 in the cavity 13 is exposed.

The measurement is performed by a measuring instrument through irradiating laser perpendicularly to the first sheet 10. In this measurement, the difference in height between the surface of the base end of the protrusion portion 12 in the heat-generating material 41 and the other surface 10b of the first sheet 10 is measured. At this time, the laser scans along the diameter direction of the surface of the base end of the protrusion portion 12 of the heat-generating material 41 (the diameter direction of the protrusion portion 12) in a manner that laser passes through the center of the surface, so that the maximum difference in height is obtained. At this time, the scanning distance is set to be longer than the diameter of the protrusion portion 12.

Here, in a case where the shape of the inner circumferential surface of the protrusion portion 12 and the shape of the outer circumferential surface of the protrusion portion 12 (the shape of the outer circumferential surface of the cavity 13) are actually same to each other, the height of the heat-generating material 41 is a value obtained by subtracting the maximum difference in height obtained by the measurement from the height of the protrusion portion 12.

The height of the protrusion portion 12 is measured according to the following method. That is, opposite to the measurement of the above-mentioned difference in height, the front surface and the back surface of the first sheet 10 is arranged upside down, and the maximum difference in height when scanning laser in a manner of passing the vertex of the protrusion portion 12 is obtained. At this time, the laser is set to scan in the diameter direction of the protrusion portion 12, and the scanning distance is set to be longer than the diameter of the protrusion portion 12.

On the other hand, when the shape of the inner circumferential surface of the protrusion portion 12 is different from the shape of the outer circumferential surface of the protrusion portion 12 (the shape of the outer circumferential surface of the cavity 13), for example, when the outer circumferential surface of the cavity 13 is in the shape of a frustum, the heat-generating material 41 filled in the cavity 13 is removed in a nitrogen atmosphere. At this time, the heat-generating material 41 is removed by using a brush and the like, while taking care not to change the shape of the cavity 13. Moreover, same as the above-mentioned measurement of the maximum difference in height, the height of the cavity 13 is measured by scanning the laser.

At least one of the first sheet 10 and the second sheet 20 has air permeability. Thus, the oxygen can be supplied to the heat-generating material 41 through at least one of the first sheet 10 and the second sheet 20, so that the heat-generating material 41 can generate heat.

In the case of the present embodiment, for example, the first sheet 10 has air permeability, and the air permeability of the first sheet 10 is higher than that of the second sheet 20.

More specifically, the first sheet 10 also has air permeability at the protrusion portion 12, and can supply oxygen to the heat-generating material 41 via the protrusion portion 12, and can release water vapor via the protrusion portion 12.

More specifically, in the case of the present embodiment, the second sheet 20 is, for example, a non-air permeable sheet through which air does not actually pass.

For example, a sheet constituted by an absorbent polymer an artificial fiber nonwoven fabric, a cellulose nonwoven fabric, paper, and the like can be used as the water absorption sheets (the first water absorption sheet 51 and the second water absorption sheet 52).

Among these, it is preferable that the water absorption sheet is constituted by an absorbent polymer, and the water absorption sheet may also be obtained by forming the absorbent polymer into a sheet shape.

It is preferable that the water absorption sheet covers at least the base end of each protrusion portion 12. In this case, the water absorption sheet covers the base end of the filled region of the heat-generating material 41 filled in each protrusion portion 12. Therefore, the moisture in the heat-generating material 41 filled in each protrusion portion 12 can be appropriately absorbed by the water absorption sheet.

In addition, in the present embodiment, the warming patch for foot 100 may also have an absorbent polymer which is discontinuously arranged (arranged in a shape other than a sheet shape) as an alternative for the water absorption sheet.

In addition, in the case of the present embodiment, it is preferable that the heat-generating material 41 does not actually contain the absorbent polymer, and with such a configuration, the ratio of the oxidizable metal in the heat-generating material 41 can be sufficiently ensured, and thus, the amount of heat generated by the heat-generating material 41 and the duration time of heat generation can be sufficiently ensured.

In addition, a peeling paper (not shown) covering the adhesive layer 61 is attached to the warming patch for foot 100 in a state before use. When the warming patch for foot 100 is used, the peeling paper can be peeled from each adhesive layer 61, and the adhesive layers 61 can be attached to the upper surface of the insole of a shoe.

Hereinafter, an example of the material and characteristics of each part of the warming patch for foot 100 will be described in more detail.

An oxidizable metal of the material which is commonly used as such a heat-generating material can be used as the oxidizable metal in the heat-generating material 41. It is preferable to use a metal in the conformation of powder or fiber as the oxidizable metal, in view of handleability, formability, and the like.

Examples of the oxidizable metal in the conformation of powder include: iron powder, aluminum powder, zinc powder, manganese powder, magnesium powder, calcium powder, and the like, among which, it is preferable to use iron powder in view of handleability and manufacturing cost.

In view that that the reaction can be well controlled, it is preferable to use a metal having a particle diameter (hereinafter, "particle diameter" means the maximum length in the conformation of powder or the mean particle diameter measured by the dynamic light scattering, laser diffractometry and the like) from 0.1 µm to 300µm, more preferable to use a metal having a particle diameter from 0.1 µm to 150 µm and having 50% by mass of metal or more, as the oxidizable metal in the conformation of powder.

In addition, examples of the oxidizable metal in the conformation of fibers include: steel fiber, aluminum fiber, magnesium fiber, etc. From the viewpoint of handleability, manufacturing cost and the like, it is preferable to use steel fibers, aluminum fibers and the like among these fibers. From the viewpoint of heat generation performance and the like, it is preferable to use a metal having a fiber length of 0.1 mm to 50 mm and a thickness of 1µm to 1000µm as the oxidizable metal in the conformation of fibers.

The content of the oxidizable metal in the heat-generating material 41 is preferably from 30% by mass to 80% by mass, more preferably from 40% by mass to 70% by mass.

Since the heat generating temperature of the protrusion portion 12 filled with heat-generating material 41 can be raised to or above an extent that a person can feel heat when touching it with a fingertip and the like, it is preferable that the content of the oxidizable metal in the heat-generating material 41 is set to be 30% by mass or more.

Sufficient air permeability of the heat-generating material 41 can be achieved by setting the content of the oxidizable metal in the heat-generating material 41 to 80% by mass or less, and as a result, a sufficient reaction can be caused in the range up to the central portion of the heat-generating material 41, and the heat generating temperature of the heat-generating material 41 can be sufficiently raised. In addition, not only a sufficient duration of heat generation of the heat-generating material 41, but also a sufficient water supply by the water retaining agent can be realized.

Here, the content of the oxidizable metal in the heat-generating material 41 can be measured by the ash content test method based on JISP8128, or, In the case that the oxidizable metal is iron, the content can be measured by a vibration sample type magnetization measurement test and the like, using the property that the iron is magnetized when an external magnetic field is applied.

A water retaining agent of the material which is commonly used for such heat-generating material can be used as the water retaining agent in the heat-generating material 41. The water retaining agent functions as a water retention agent. In addition, the water retaining agent may also have the following function of supplying agent: maintaining oxygen supplied to the oxidizable metal and supplying the oxygen to the oxidizable metal.

It is preferable to use, for example, a water retaining agent of an inorganic material as the water retaining agent.

It is preferable to use, for example, a porous material as the water retaining agent.

Examples of the water retaining agent may include: activated carbon (coconut shell carbon, charcoal powder, pitch carbon, peat, brown carbon), carbon black, acetylene black, graphite, zeolite, pearlite, vermiculite, silica, cancrinite, fluorite, and the like, among which, it is preferable to use activated carbon, from the viewpoint of having water retention performance, oxygen supply performance, and catalytic performance.

In view of being able to form an effective contact state with the oxidizable metal, it is preferable to use a water retaining agent in a form of powder having a particle diameter of 0.1 µm to 500 µm, and more preferable to use a water retaining agent in a form of powder having a particle diameter of 0.1µm to 200 µm and 50% by mass and more as the water retaining agent.

A water retaining agent having the form other than the above-mentioned powder may alternatively be used, and for example, a water retaining agent having the fibrous form may be used as the water retaining agent.

The content of the water retaining agent in the heat-generating material 41 is preferably from 1% by mass to 50% by mass, more preferably from 2% by mass to 40% by mass.

By setting the content of the water retaining agent in the heat-generating material 41 to 1% by mass or more, water can be sufficiently accumulated in the heat-generating material 41, and the oxidation reaction of the oxidizable metal can be continued to or above an extent that the temperature thereof rises to the human body temperature or higher. In addition, since the air permeability of the heating material 41 is sufficiently ensured, the oxygen can be sufficiently supplied to the heating-material 41, and the heating efficiency of the heat-generating material 41 can be improved.

By setting the content of the water retaining agent in the heat-generating material 41 to 50% by mass or less, the heat capacity of the heat-generating material 41 can be suppressed with respect to the obtained amount of heat generation, so that the heat-generating temperature rise becomes larger, and a temperature rise making a person to feel warm becomes larger.

The heat-generating material 41 may also contain an electrolyte.

An electrolyte of a material which is commonly used for such a heat-generating material can be used as the electrolyte.

Examples of the electrolyte may include: chlorides or hydroxides of alkali metals, rare earth metals or heavy metals, etc. Moreover, among these, it is preferable to use various chlorides such as sodium chloride, potassium chloride, calcium chloride, magnesium chloride, and ferric chloride (divalent or trivalent), from the viewpoint of conductivity, chemical stability, and excellent production cost, These electrolytes may be used alone or in combination of two or more.

For the content of the electrolyte in the heat-generating material 41, the mass ratio of water in the heat-generating material 41 is preferably from 0.5% by mass to 24% by mass, more preferably form 1% by mass to 10% by mass.

By setting the content to be 0.5% by mass or more, the oxidation reaction of the heat-generating material 41 can be sufficiently carried out, and the electrolyte required by the heat-generating function can be secured. Therefore, the moisture ratio of the heat-generating material 41 can also be suppressed, and as a result, the rise of the heat-generating temperature can be sufficiently secured.

By setting the content to be 24% by mass or less, the air permeability of the heat-generating material 41 can be improved, and the electrolyte required by the heat-generating function can be secured. Therefore, the moisture ratio of the heat-generating material 41 can be maintained at a certain level, sufficient water can be supplied to the oxidizable metal and the like, heat-generating performance is excellent, and the electrolyte can be uniformly blended in the heat-generating material 41. Thus, the content of 24% by mass or less is preferable.

In addition, a thickening agent and a coagulant may be added to the heat-generating material 41, and other additives may also be added.

In order to uniformly fill the cavities 13 of the fine protrusion portion 12 with the heat-generating material 41, it is preferable that the heat-generating material 41 is filled in the cavities 13 in a slurry state where fluidity is maintained, and in this case, the heat-generating material 41 is preferable to contain a thickening agent. A substance whose consistency is increased or to which a thixotropic property is provided by absorbing water, for example, a water-soluble polymer material can be used as the thickening agent.

The temperature reached by the heat generation of the protrusion portion 12 in the warming patch for foot 100 is preferably from 35 °C to 98 °C and more preferably from 38 °C to 70 °C.

The temperature reached by the heat generation of the warming patch for foot 100 can be measured by a method equivalent to JIS S4100.

For the first sheet 10 in which the protrusion portion 12 is filled with the heat-generating material 41, the amount of water vapor generated per unit weight (1 g) of the heat-generating material 41 within 10 minutes is preferably from 20 mg/g to 250 mg/g, and more preferably form 70 mg/g to 180 mg/g.

Here, the water vapor amount (water vapor generation amount) is measured according to, for example, the following method.

The measuring device comprises: an aluminum-made measuring chamber (volume: 4.2 L), an inflow passage through which dehumidified air (humidity: less than 2%, flow rate: 2.1 L/min) flows into the lower section of the measuring chamber, and an outflow passage through which air flows out from the upper section of the measuring chamber. An inlet psychrometer and an inlet flow meter are installed in the inflow passage. On the other hand, an outlet psychrometer and an outlet flow meter are installed in the outflow passage. A thermometer (thermistor) is installed in the measuring chamber. A thermometer having a temperature resolution of 0.1°C degree is used as the thermometer.

The warming patch for foot 100 is taken out of the package at a measuring ambient temperature of 30°C (30 ± 1°C), and then placed in the measuring chamber with the surface 10a of the first sheet 10 facing upward, and a thermometer provided with a metal ball (mass 4.5 g) is placed on thereon. In this state, dehumidified air flows from the lower section of the measuring chamber, and the difference in absolute humidity before and after the air flows into the measuring chamber is obtained based on the temperature and humidity measured by the inlet hygrometer and the outlet hygrometer. In addition, the amount of water vapor released from the warming patch for foot 100 is calculated based on the flow rate measured by the inlet flow meter and the outlet flow meter. The amount of water vapor generated from the start of the measurement to 10 minutes later is measured.

Examples of the material of the nonwoven fabric sheet constituting the first sheet 10 may include synthetic fibers, natural fibers, or composite fibers thereof, and examples of the production method may include: spunbonded method, needled method, spunlaced method, melt blown method, flash spun method, airlaid method, ventilated method, etc.

In the case of the present embodiment, the nonwoven fabric sheet constituting the first sheet 10 includes fibers constituted by a first resin material and bonding portions for bonding fibers constituted by a second resin material to each other.

There is no particular limit to the first resin material constituting the nonwoven fabric sheet and further constituting the first sheet 10, and examples thereof may include: polyethylene, polypropylene, nylon, rayon, polystyrene, acrylic acid, vinylon, cellulose, aramid, polyvinyl alcohol, polyethylene naphthalate, or polyethylene terephthalate, among which polyethylene terephthalate (PET) is preferable.

There is no particular limit to the second resin material constituting the nonwoven fabric sheet and further constituting the first sheet 10, and it is preferable a material whose melting point lower than that of the first resin material constituting the nonwoven fabric sheet. Examples of the second resin material constituting the nonwoven fabric sheet may include: polyethylene, polypropylene, ethylene vinyl acetate resin, or low melting point PET (copolyester), among which polyethylene or low melting point PET is preferable.

In addition, the fibers constituting the nonwoven fabric sheet may also be in a core-sheath structure including a core constituted by the first resin material and a sheath constituted by the second resin material.

The first sheet 10 and the nonwoven fabric sheet may further include a second bonding portion that constituted by at least one resin material having a melting point lower than that of the first resin material and higher than that of the second resin material, and in the bonding portion the at least one resin material bonds fibers of a resin material having a higher melting point (a group of unmelted resins (including at least the first resin material) during the processing of the nonwoven fabrics while forming the protrusion portion 12) to each other.

The content of the first resin material in the nonwoven fabric sheet constituting the first sheet 10 is more than the content of the second resin material in the nonwoven fabric sheet.

The content of the first resin material in the nonwoven fabric sheet is preferably from 60% by mass to 95% by mass. In addition, the content of the second resin material in the nonwoven fabric sheet is from 5% by mass to 40% by mass.

It is possible to sufficiently ensure the air permeability of the nonwoven fabric sheet and the stiffness of the nonwoven fabric sheet by setting the contents of the first resin material and the second resin material in the nonwoven fabric sheet in such a manner.

The weight per unit area of the nonwoven fabric sheet constituting the first sheet 10 is preferably from 15 g/m² to 500 g/m², and particularly preferably from 30 g/m² to 350 g/m². By setting the weight per unit area of the nonwoven fabric sheet to 15 g/m² or more, not only the sufficient stiffness of the first sheet 10 can be ensured, but also the temperature of the heat-generating material 41 can be appropriately eased and then transferred to skin. By setting the weight per unit area of the nonwoven fabric sheet to 500 g/m² or less, the temperature of the heat-generating material 41 can be efficiently transferred to skin via the first sheet 10.

The thickness of the base portion 11 of the first sheet 10 is preferably from 0.03 mm to 2.6 mm, and particularly preferably from 0.08 mm to 1.25 mm. By setting the thickness of the base portion 11 to 0.03 mm or more, the form retention of the first sheet 10 (in particular, the form retention of the protrusion portion 12) can be achieved, and further, the good form retention of the warming patch for foot 100 can be achieved. By setting the thickness of the base portion 11 to 2.6 mm or less, a good heat transfer property of the first sheet 10 can be achieved.

The moisture permeability of the first sheet 10 is preferably, for example, from 1000 g/(m²·24h) to 17000 g/(m²·24h), and more preferably from 2000 g/(m²·24h) to 12000 g/(m²·24h).

In the case of the present embodiment, the moisture permeability of the second sheet 20 is lower than the moisture permeability of the first sheet 10.

The moisture permeability of the second sheet 20 is preferably, for example, 2000 g/(m²·24h) or less, particularly preferably 1000 g/(m²·24h) or less.

By setting the moisture permeability of the second sheet 20 in such a range, the generation direction of the water vapor while the heat-generating material generates heat 41 can be limited by the second sheet 20. For example, by supplying oxygen to the heat-generating material 41 from the first sheet 10, the generation of water vapor from the second sheet 20 can be suppressed, and water vapor can be mainly generated from the first sheet 10.

The weight per unit area of the second sheet 20 is from 10 g/m² to 200 g/m², and preferably from 20 g/m² to 100 g/m². By setting the weight per unit area of the second sheet 20 in such a range, the generation direction of the water vapor while the heat is generated can be limited by the second sheet 20.

Examples of the second sheet 20 may include a sheet of a resin film constituting by a resin of polyolefin such as polyethylene and polypropylene, polyester, polyamide, polyurethane, polystyrene, nylon, polyvinylidene chloride, a copolymer of polyethylene-vinyl acetate, and the like. In particular, by using a sheet in which inorganic filler such as titanium oxide is blended with the above-mentioned resin, a good shielding property of the heat-generating material 41 can be achieved by second sheet 20. A plurality of second sheets 20 may also be used in overlapping.

More specifically, examples of the second sheet 20 may include: a laminated sheet of paper and the above-mentioned resin film, or a laminated sheet of nonwoven fabrics and the above-mentioned resin film. In this case, the resin film is formed to be the inner surface (water absorption sheet) of the second sheet 20, and the paper or nonwoven fabrics constituting the second sheet 20 is disposed on the outer surface of the second sheet 20. In addition, it is also possible to laminate nonwoven fabrics on surface of the second sheet 20 facing the third sheet 30 in order to suppress heat release to the outer surface.

In the case that the water absorption sheets (the first water absorption sheet 51, the second water absorption sheet 52) is an absorbent polymer sheet, the absorbent polymer constituting the absorbent polymer sheet is a polymer particle having water absorbability.

There is no particular limit to the shape of the absorbent polymer, and it may be spherical, massive, botryoidal, indefinite, porous, powdery, or fibrous. The mean particle diameter of the absorbent polymer may be set to be from 100 µm to 1000 µm, preferably from 150 µm to 650 µm, and more preferably 200 µm to 500 µm, in order to suppress the detachment of the absorbent polymer from the warming patch for foot 100 and the movement of the absorbent polymer.

As an example, in order to obtain the absorbent polymer, one or more monomers selected from the following monomers will be polymerized and, if necessary, crosslinked. There is no particular limit of the polymerization method herein, and various commonly known polymerization methods of absorbent polymers, such as reverse suspension polymerization and aqueous solution polymerization and the like, can be employed. Moreover, if necessary, the polymer obtained by polymerization is subjected to a treatment such as milling and classification, so that the polymer is adjusted to have a desired mean particle diameter, and if necessary, the polymer is treated with inorganic fine particles, so that an absorbent polymer is obtained.

A monomer of an unsaturated group having water-solubility and polymerism can be employed as the monomer used to produce the absorbent polymer. Specific examples of such monomers may include: an ethylene monomer of an unsaturated group having polymerism including an olefin unsaturated carboxylic acid or a salt thereof, an olefin unsaturated carboxylic acid ester, an olefin unsaturated sulfonic acid or a salt thereof, an olefin unsaturated phosphoric acid or a salt thereof, an olefin unsaturated phosphoric acid ester, an olefin unsaturated amine, an olefin unsaturated ammonium salt, an olefin unsaturated amide, and the like.

There is no particular limit to the thickness of the water absorption sheets (the first water absorption sheet 51 and the second water absorption sheet 52), and for example, it may be from 0.05 mm to 2 mm, and preferably from 0.1 mm to 1 mm.

By setting the thickness of the water absorption sheet to be 0.05 mm or more, water can be sufficiently absorbed by the water absorption sheet. In addition, the warming patch for foot 100 can be sufficiently configured as a sheet type by setting the thickness of the water absorption sheet to be 2 mm or less.

The measurement of the thickness of the water absorption sheet can be performed according to, for example, the measurement method with Peacock meter.

A thin sheet material capable of absorbing and holding water and having flexibility can be used as the water absorption sheets (the first water absorption sheet 51 and the second water absorption sheet 52). Examples of such thin sheet materials may include: paper with fiber as raw material, a fiber sheet such as nonwoven fabrics, textiles, and knitted fabrics and the like, and a porous body such as a sponge and the like. Examples of fibers to be used as the material of the water absorption sheet 40 may include: fibers with natural fibers such as plant fibers or animal fibers as main ingredient, or fibers with chemical fibers as main ingredient. Examples of the plant fibers may include one or more selected form cotton, kapok, wood pulp, non-wood pulp, peanut protein fiber, corn protein fiber, soybean protein fiber, mannan fiber, rubber fiber, hemp, Manila hemp, sisal, New Zealand flax, kender, coconut, rush, and straw. Examples of the animal fibers may include one or more selected from a group constituted by sheep wool, goat hair, mohair, cashmere, alpaca hair, Angora goat hair, camel hair, vicuna hair, silk, goose feather, goose down, feather, alginate fiber, chitin fiber, and casein fiber. For example, one or more of man-made fibers, acetate fibers and cellulose can be used as the chemical fibers.

The water absorption sheets (the first water absorption sheet 51 and the second water absorption sheet 52) is preferably a sheet containing an absorbent polymer and a fiber material constituted by the above-mentioned fibers.

When the water absorption sheet contains a component (a) of the fibrous material and a component (b) of the absorbent polymer (b), examples of the forms of the water absorption sheet may include the following three forms: (i) a one-sheet form in the state where the component (a) and the component (b) are uniformly mixed, (ii) a form in which the component (b) is disposed between the same or different sheets containing the component (a), and (iii) a flake form in which the component (b) is dispersed.

Among these, the preferable sheet is a sheet of the form (ii). In addition, the water absorption sheet in the form of (ii) can be produced by the following method, for example: uniformly dispersing the component (b) of the absorbent polymer on a sheet containing the component (a), after spraying water in an amount of 200 g/m² from above, further laminating the same or different sheets containing the component (a) above the sheet, and stamping and drying the sheet is at 100 ± 0.5°C under a pressure of 5 kg/cm² until the sheet is dried to have a water content of 5% by mass or less.

The sufficient water absorption performance of the water absorption sheets (the first water absorption sheet 51 and the second water absorption sheet 52) can be ensured by using a hydrogel material capable of absorbing and holding a liquid 20 times or more of its own weight as the absorbent polymer. Examples of the particle shape of the absorbent polymer may include: spherical shape, massive shape, botryoidal shape, fibrous shape and the like. The particle diameter of the absorbent polymer particles is preferably 1 µm or more, and more preferably 10 µm or more. In addition, the particle diameter of the absorbent polymer particles is preferably 1000 µm or less, and more preferably 500 µm or less. In addition, the particle diameter of the absorbent polymer particles is measured by a dynamic light scattering method, a laser diffractometry method and the like.

Specific examples of the absorbent polymer may include one or more selected from a group constituted by starch, cross-linked carboxymethylated cellulose, polymers or copolymers of acrylic acid or alkali metal salts of acrylic acid, polyacrylic acid and salts thereof, and polyacrylate graft polymers. Among them, it is preferable to use a polymer or copolymer of acrylic acid or an alkali metal salt of acrylic acid, polyacrylic acid or a salt thereof, or polyacrylate graft polymers, since the water absorption performance of the water absorption sheets (the first water absorption sheet 51 and the second water absorption sheet 52) can be improved.

The ratio of the particles of the component (b) of the absorbent polymer in the water absorption sheets (the first water-absorbent sheet 51 and the second water-absorbent sheet 52) is preferably 10% by mass or more, and more preferably 20% by mass or more, measured in the dry state. In addition, the ratio of the particles of the component (b) of the absorbent polymer in the water absorption sheets is preferably 70% by mass or less, and more preferably 65% by mass or less, measured in the dry state.

The weight per unit area of the water absorption sheets (the first water absorption sheet 51 and the second water absorption sheet 52) in the dry state is preferably from 20 g/m² to 250 g/m², more preferably from 40 g/m² to 220 g/m², and further preferably from 60 g/m² to 180 g/m². The weight per unit area of the component (b) contained in the water absorption sheet 40 in a dry state is preferably from 5 g/m² to 200 g/m², more preferably from 10 g/m² to 170 g/m², and further preferably from 30 g/m² to 130 g/m².

In the case of the present embodiment, a water absorption sheet which is integrated by laminating paper made of wood pulp (weight per unit area: 20 g/m²) and an absorbent polymer (spherical, mean particle diameter: 300 µm, weight per unit area: 90 g/m²) and paper made of wood pulp (weight per unit area: 30 g/m²), can be used as the water absorption sheets (the first water absorption sheet 51 and the second water absorption sheet 52).

A nonwoven fabric sheet same as that of the first sheet 10 can be used as the material of the third sheet 30. In addition, the thickness of the third sheet 30 is preferably larger than the thickness of the first sheet 10.

There is no particular limit to the material of the adhesive layer 61, and for example, adhesive materials such as rubber, acrylic, silicone, emulsion, hot melt, hydrogel and the like can be used.

In the case of the present embodiment, the warming patch for foot 100 is designed to be left-right symmetrical and can be used for any foot of the left or the right foot.

The warming patch for foot 100 of the present embodiment is preferably a set of two sheets or a set of a plurality of sheets (preferably a set of an even number of sheets), and sheets are bundled and then circulated (for sale and the like).

However, the invention is not limited to this example, and the arrangement manner of the protrusion portion 12 of the warming patch for foot 100 may also be different when used for the right foot and the left foot.

Here, a manufacturing method of the warming patch for foot 100 will be briefly described.

When the protrusion portion 12 is formed on the first sheet 10 by pressing, a nonwoven fabric sheet to be the material of the first sheet 10 is first prepared. The nonwoven fabric sheet includes, for example, a first fiber constituted by a first resin material and a second fiber constituted by a second resin material (in the case of cotton mixing the first fiber and the second fiber). In addition, the fibers constituting the nonwoven fabric sheet may also be in a core-sheath structure including a core constituted by the first resin material and a sheath constituted by the second resin material.

Then, the first sheet 10 on which the protrusion portion 12 is formed is formed by hot stamping the nonwoven fabric sheet.

Here, the temperature of the hot stamping is set to be a temperature between the melting point of the first resin material and the melting point of the second resin material. That is, the temperature of the hot stamping is set to: a temperature lower than the melting point of the first resin material and higher than the melting point of the second resin material.

Thus, the second resin material is melted while the first resin material is not melted, so the fibers constituted by the first resin material (the fibers may also be core portions of the core-sheath structure) are bonded to each other by the melted second resin material. That is, a bonding portion is formed, in which the fibers constituted by the first resin material are bonded to each other by the melted second resin material.

As a result, the air permeability of the first sheet 10 is ensured, and the stiffness of the first sheet 10 is also sufficiently ensured. That is, the air permeability and the stiffness of the base portion 11 can be sufficiently ensured, and for the protrusion portion 12, the air permeability from the base end to the top end of the protrusion portion 12 can also be ensured, and the entire protrusion portion 12 can be set to have sufficient stiffness.

After the protrusion portion 12 is formed on the first sheet 10 in this manner, the protrusion portion 12 is filled with the heat-generating material 41.

To this end, a raw material composition (slurry material) of the heat-generating material 41 containing the oxidizable metal, the water retaining agent, and water is prepared, and the raw material composition is made to flow into each protrusion portion 12.

Thus, the heat-generating material 41 can be filled along the shape of the inner peripheral surface of the protrusion portion 12.

After the heat-generating material 41 is filled in each protrusion portion 12 of the first sheet 10, the water absorption sheets (the first water absorption sheet 51 and the second water absorption sheet 52) are laminated on the base portion 11 of the first sheet 10.

Further, the second sheet 20 is laminated on the water absorption sheet, and the first portion 11a of the base portion 11 of the first sheet 10 and the second sheet 20 are joined to each other.

An adhesive may be used to join the second sheet 20 to the first sheet 10, or the joining may also be performed by heat sealing.

Further, the third sheet 30 is laminated on the second sheet 20, and the second portion 30b of the third sheet 30 and the second sheet 20 are joined to each other.

In this way, the warming patch for foot 100 can be produced.

Next, the method of using warming patch for foot of this embodiment will be explained.

In this use method, the above-mentioned warming patch for foot 100 is used to press skin of the foot sole region corresponding to the Yongquan acupuncture point 91 and skin of the foot sole region corresponding to the insomnia acupuncture point 92 through the plurality of protrusion portions 12, respectively.

Thus, it can be expected that the fatigue of the feet can be eliminated and the sleep quality can be improved.

In the present embodiment, the Yongquan acupuncture point 91 can be pressed by any one of the plurality of first protrusion portions 12a, and the insomnia acupuncture point 92 can be pressed by any one of the plurality of second protrusion portions 12b.

More specifically, in a state where the warming patch for foot 100 is attached to the upper surface of the insole of a shoe by using the adhesive layer 61 of the warming patch for foot 100, the user can arrange the warm paste 100 below the foot sole by putting on the shoes. In this state, when the user stands up, walks and the like, stimulation can be performed by pressing skin of the foot sole with the protrusion portion 12.

More specifically, for example, it is preferable to start the pressing with the plurality of protrusion portions 12 of the warming patch for foot 100 30 minutes to 120 minutes before bedtime, and to stop the pressing before bedtime. Here, "start the pressing" means, for example, standing up or walking in a state in which the warming patch for foot 100 is arranged below the foot sole. In addition, "stop the pressing" means that the warming patch for foot 100 is removed from the foot sole (e.g., taking off the shoes mounted with the warming patch for foot 100) and the like.

In this way, it can be expected that the sleep quality is improved by using the warming patch for foot 100 before bedtime.

### <Modification of first embodiment>

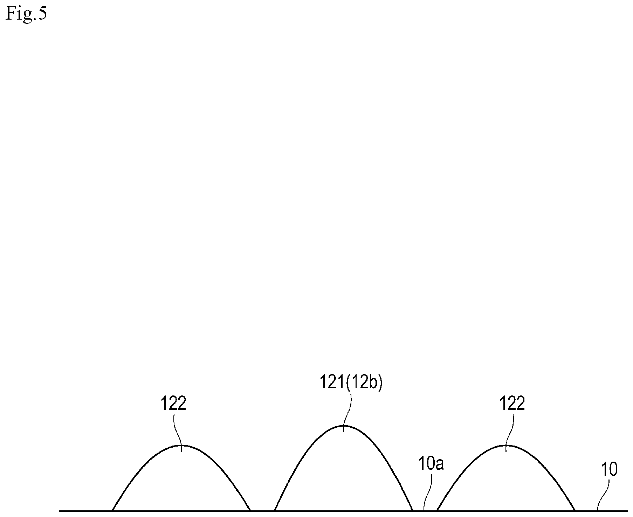

Next, a modified example of the first embodiment will be described with reference to Fig. 5.

As shown in Fig. 5, in the case of the modified example, the height of the central protrusion portion 121 is higher than the height of the peripheral protrusion portion 122.

With this structure, the insomnia acupuncture points 92 can be pressed with more sufficient pressing force through the central protrusion portion 121.

### (Second embodiment)

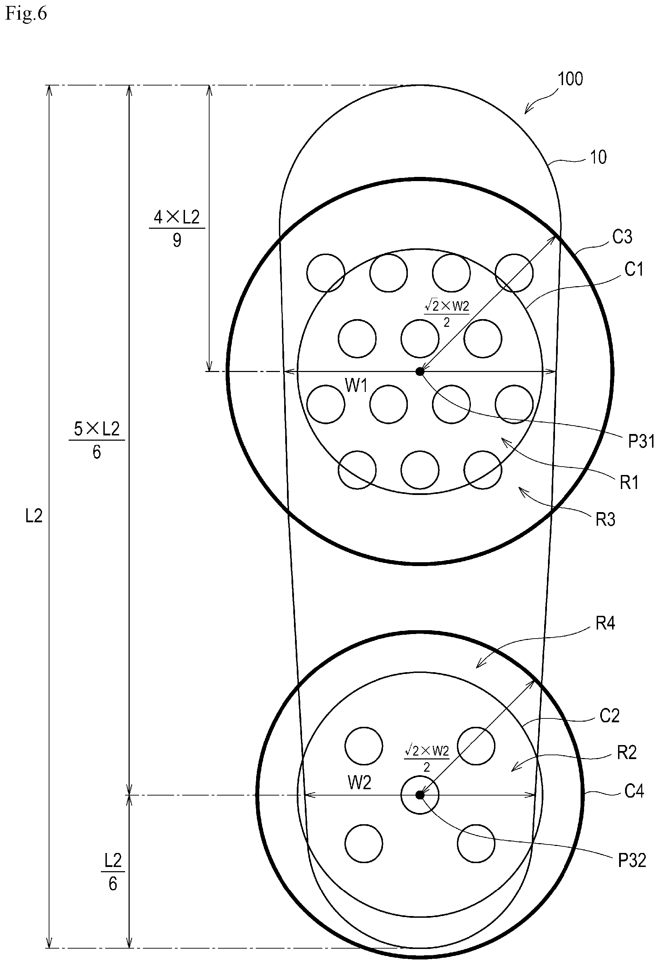

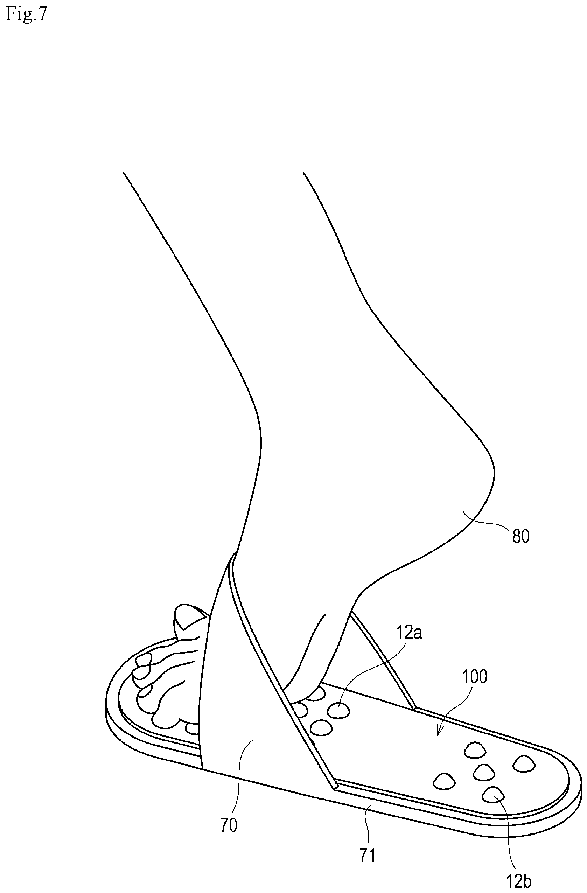

Next, a second embodiment will be described with reference to Figs. 6 and 7.

Compared with the warming patch for foot 100 according to the above-mentioned first embodiment described above, the warming patch for foot 100 according to the present embodiment has differences in the following aspects, and has same configurations in the other aspects.

In the present embodiment, the warming patch for foot 100 is also designed in a shape of a human footprint.

In the above-mentioned first embodiment, the warming patch for foot 100 without a portion for placing toes has been described. In contrast, in the present embodiment, a warming patch for foot 100 with a portion for placing toes will be described. Therefore, compared with the warming patch for foot 100 of the above-mentioned first embodiment described above, the warming patch for foot 100 of the present embodiment is designed in a shape of strip in the front-rear direction.

As shown in Fig. 6, in the present embodiment, the warming patch for foot 100 is also designed in a shape of a human footprint.

In the case of the present embodiment, when the length of the warming patch for foot 100 (the dimension of the warming patch for foot 100 in the front-rear direction) is set to be L2, the plurality of protrusion portions 12 includes: a first protrusion portion 12a disposed in a first circular region R1 (a region within a circle C1 shown in Fig. 6), which is centered on the width center P31 at 4×L2/9 from the tiptoe toward the heel of the warming patch for foot 100 and has a radius of 3 cm, and a second protrusion portion 12b disposed in a second circular region R2 (a region within a circle C2 shown in Fig. 6), which is centered on the width center P32 at 5×L2/6 from the tiptoe toward the heel of the warming patch for foot 100 and has a radius of 3 cm.

In addition, the plurality of first protrusion portions 12a are disposed in: a range of ±3 cm in the longitudinal direction (front-rear direction) of the warming patch for foot 100, taken the position 4×L2/9 from the tiptoe toward the heel of the warming patch for foot 100 as a reference.

In addition, the plurality of second protrusion portions 12b are disposed in: a range of ±3 cm in the longitudinal direction of the warming patch for foot 100, taken the position 5×L2/6 from the tiptoe toward the heel of the warming patch for foot 100 as a reference.

Here, the width of the warming patch for foot at 4×L2/9 from the tiptoe toward the heel of the warming patch for foot 100 is set to be W1, and the width of the warming patch for foot at 5×L2/6 from the tiptoe toward the heel of the warming patch for foot is set to be W2.

The plurality of protrusion portions 12 includes: a first protrusion portion 12a disposed in a third circular region R3 (a region within a circle C3 shown in Fig. 6), which is centered on the width center P31 at 4×L2/9 from the tiptoe toward the heel of the warming patch for foot 100 and has an area S3 of (W1)²×π/2, and a second protrusion portion 12b disposed in a fourth circular region R4 (a region within a circle C4 shown in Fig. 6), which is centered on the width center P32 at 5×L2/6 from the tiptoe toward the heel of the warming patch for foot 100 and has an area S4 of (W2)²×π/2.

In the case of the present embodiment, in the warming patch for foot 100, a region that does not belong to either of the third circular region R3 or the fourth circular region R4 is a non-formed region in which the protrusion portion 12 is not formed.

In addition, the same limitations as those in the first embodiment can also be applied to the arrangement of the first protrusion portion12a and the protrusion portion 12b in this embodiment.

That is, a region that does not belong to any one of "a circular region which is centered on the width center P23 at L1/3 from the middle portion P21 toward the heel and has an area of (W1)²×π/2, a circular region which is centered on the width center P24 at 4×L1/5 from the middle portion P21 toward the heel and has an area of (W2)²×π/2, a circular region which is centered on the width center P31 at 4×L2/9 from the tiptoe toward the heel of the warming patch for foot 100 and has an area of (W1)²×π/2, a circular region which is centered on the width center P32 at 5×L2/6 from the tiptoe toward the heel of the warming patch for foot 100 and has an area of (W2)²×π/2" is a non-formed region in which the protrusion portion 12 is not formed.

Figure 7 shows that: the warming patch for foot 100 of the present embodiment is attached to the upper surface of the insole 71 of a shoe 70, and the shoe 70 is worn on the foot 80 of the user, whereby the warming patch for foot 100 is arranged below the foot sole.

### (Third embodiment)

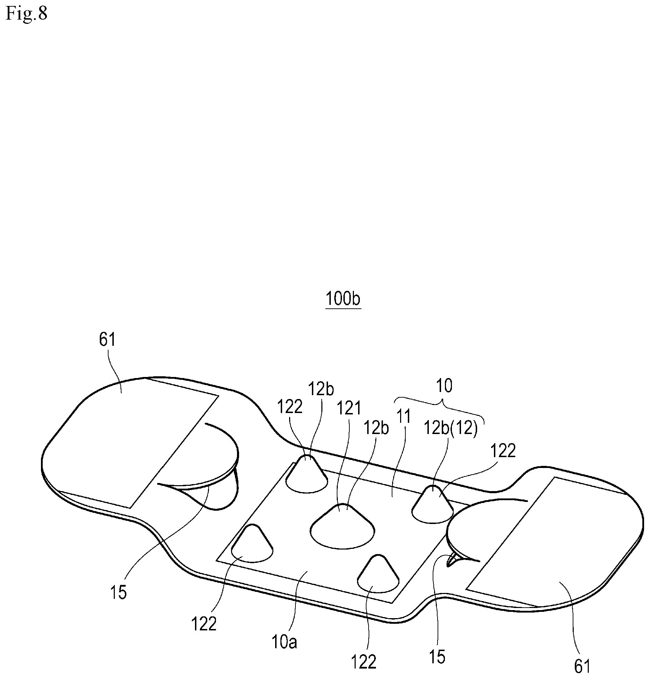

Next, a third embodiment will be described with reference to Figs. 8 to 12.

Compared with the warming patch for foot 100 according to the above-mentioned first embodiment, the warming patch for foot 100 of the present embodiment has differences in the following aspects, and has same configurations in the other aspects.

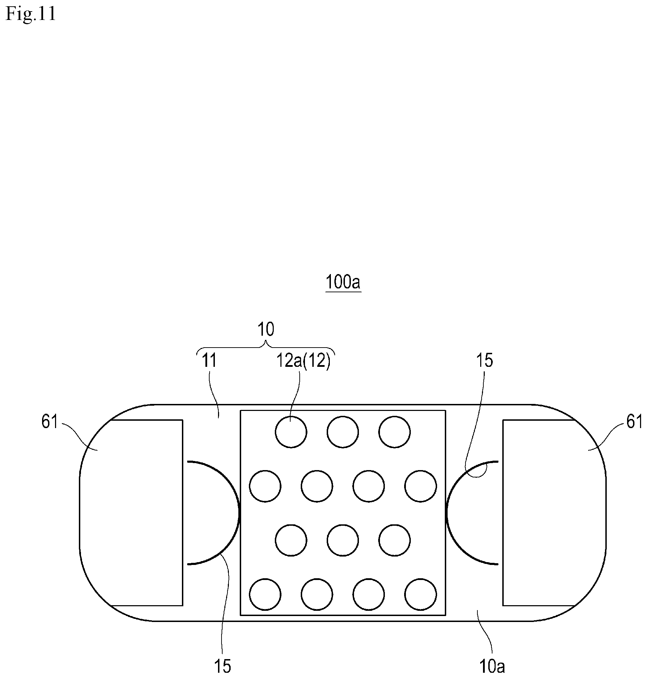

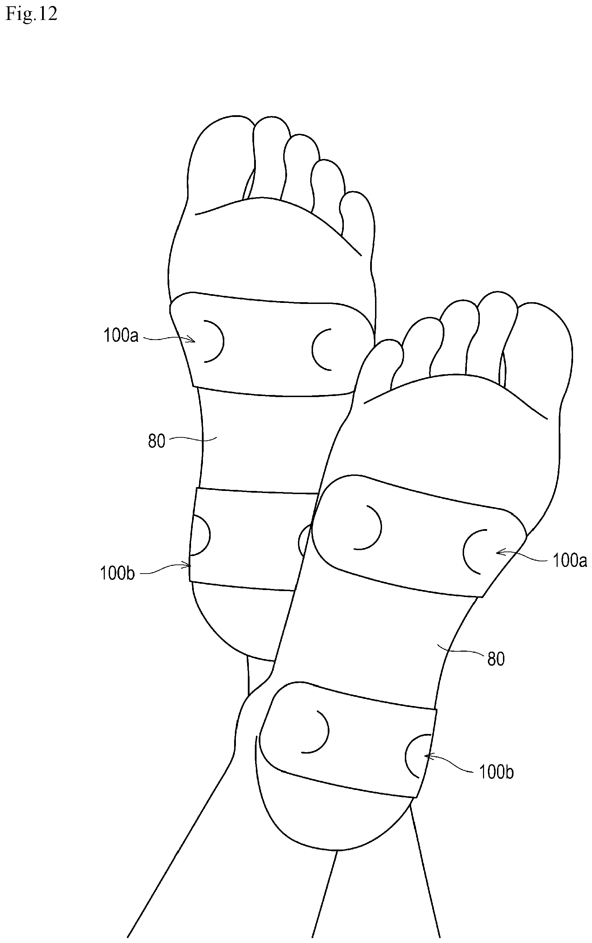

The warming patch for foot of the present embodiment is provided with: the first sheet 100a (Figs. 11 and 12) arranged on the front part of the foot sole, and the second sheet 100b (Figs. 8 to 10 and 12) arranged on the rear part of the foot sole in a state separated from the first sheet 100a.

In the present embodiment, a base material sheet includes the first base material sheet (the first sheet 10) constituting the first sheet 100a and the second base material sheet (the first sheet 10) constituting the second sheet 100b, and a plurality of protrusion portions 12 includes the first protrusion portion 12a provided in the first sheet 100a and the second protrusion portion 12b provided in the second sheet 100b.

Thus, for example, the Yongquan acupuncture point can be pressed by the first protrusion portion 12a of the first sheet 100a, and the insomnia acupuncture point can be pressed by the second protrusion portion 12b of the second sheet 100b.

Here, in a typical case, the first sheet 100a and the second sheet 100b are designed to be separated from each other from the beginning. However, the invention is not limited to this example, and the user may separate the first sheet 100a and the second sheet 100b from each other before use, and separately attach them to the front and rear portions of the foot sole for use.

Since the warming patch for foot of the present embodiment includes the nonwoven fabric, the user can stretch it a little by tensing and the like. Thus, the user can finely adjust the position of the protrusion portion 12 to fit the acupuncture point of the user.

In the case of the present embodiment, the first sheet 100a and the second sheet are used separately, so that the position can be adjusted according to the size of the foot sole of the user. That is, the protrusion for foot is usually arranged on a sheet having the same size as the foot sole, so that the protrusions for foot in different sizes have to be prepared according to the size of the foot, which is very inconvenient. However, the warming patch for foot of the present embodiment is applicable to any foot size, and is economical and practical.

In the case of the present embodiment, it is preferable to bundle and circulate the first sheet 100a and the second sheet 100b.

The warming patch for foot is preferably constituted by a plurality set of "the first sheets 100a and the second sheets 100b". In the case of the present embodiment, it is preferable to bundle and circulate the plurality sets of the first sheets 100a and the second sheets 100b.

As shown in Figs. 8 to 10, in the case of the present embodiment, the second sheet 100b has a plurality of second protrusion portions 12b. The plurality of second protrusion portions 12b includes: a central protrusion portion 121 disposed at the center of the second sheet 100b in the width direction, and a plurality of peripheral protrusion portions 122 disposed on a circumference with the central protrusion portion 121 as the center.

Here, "the width direction of the second Sheet 100b" refers a direction extending in the width direction of the foot when the second sheet 100b is attached to the foot sole for use. In the present embodiment, the longitudinal direction of the second sheet 100b becomes the width direction of the second sheet 100b, as shown in Fig. 12.

In the case of the present embodiment, in view of stimulating acupuncture points, it is preferable that the size (at least one of the height and the plane area) of the central protrusion portion 121 is equal to or larger than that of the peripheral protrusion portion 122.

On the other hand, it is preferable that the size of the central protrusion portion 121 (at least any one of the height and the plane area) is equal to or smaller than that of the peripheral protrusion portion 122, in view of uniformly transferring the heat from the plurality of second protrusion portions 12b to skin and appropriately suppressing the heat transfer to the center of the arrangement region of the plurality of second protrusion portions 12b.

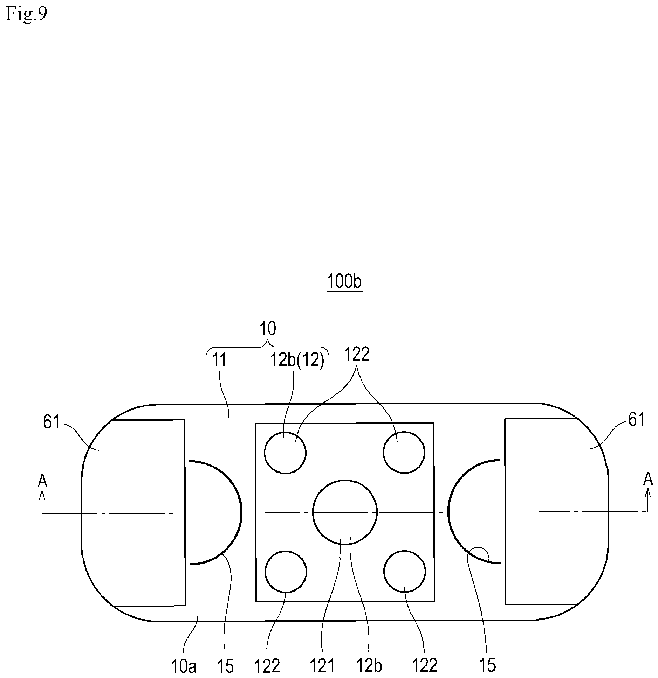

As shown in Fig. 9, in the case of the present embodiment, for example, the plane area of the central protrusion portion 121 is larger than that of the peripheral protrusion portion 122. Therefore, the insomnia acupuncture point can be pressed more stably by the central protrusion portion 121.

However, the invention is not limited to this example, and the plane areas of the plurality of second protrusion portions 12b of the second sheet 100b may be also equal to each other, or the plane area of the peripheral protrusion portion 122 may also be larger that of the central protrusion portion 121.

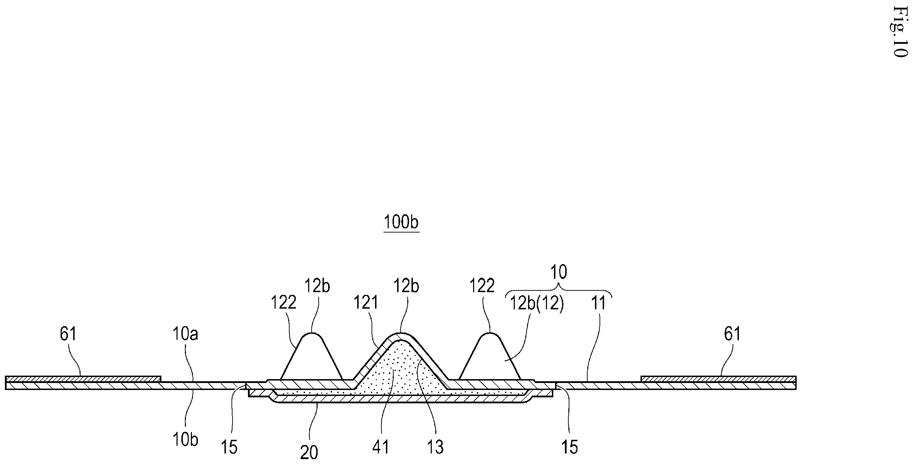

As shown in Fig. 10, in the case of the modified example, the height of the central protrusion portion 121 and the height of the peripheral protrusion portion 122 are equal to each other.

However, the invention is not limited to this example, and the height of the central protrusion portion 121 may also be higher than that of the peripheral protrusion portion 122. With this structure, the insomnia acupuncture point can be pressed with more sufficient pressing force through the central protrusion portion 121.

Alternatively, on the contrary, the height of the peripheral protrusion portion 122 may also be higher than that of the central protrusion portion 121.

As shown in Fig. 10, the second sheet 100b includes, for example, the first sheet 10 and the second sheet 20.

The second sheet 20 may be in a single-layer structure or a multi-layer structure. The second sheet 20 is disposed with a range including the arrangement region of the plurality of protrusion portions 12. In the case of the present embodiment, as shown in Fig. 10, the second sheet 20 is not disposed at both ends of the second sheet 100b in the longitudinal direction.

As an example, as shown in Fig. 10, the heat-generating material 41 is filled in a portion corresponding to the protrusion portion 12 of the first sheet 10, and besides the heat-generating material 41 is also filled between the first sheet 10 and the second sheet 20 in a portion corresponding to the base portion 11.

In addition, the illustration of the cross-sectional structure of the first sheet 100a is omitted, and the first sheet 100a has the same layer structure as the second Sheet 100b. In addition, the plane shape of the first sheet 100a is designed to be, for example, same as the plane shape of the second sheet 100b (see Figs. 11 and 12).

As shown in Fig. 11, the first sheet 100a is respectively provided with a pair of attachment portions (adhesive layers 61) at both ends of the first sheet 100a in the width direction, for attaching the first sheet 100a to the foot sole. In the first sheet 100a, a plurality of first protrusion portions 12a are arranged in a region between the pair of attachment portions.

Similarly, as shown in Fig. 8 to 10, the second sheet 100b is respectively provided with a pair of attachment portions (adhesive layers 61) at both ends of the second sheet 100b in the width direction, for attaching the second sheet 100b to the foot sole. In the second sheet 100b, a plurality of second protrusion portions 12b are arranged in a region between the pair of attachment portions.

Here, the first sheet 100a and the second sheet 100b of the present embodiment may also be a warming patch for foot, respectively.

That is, in the case of the present embodiment, the warming patch for foot is provided with a pair of attachment portions (adhesive layers 61) at both ends of the warming patch for foot in the width direction for attaching the warming patch for foot to the foot sole, respectively, and the plurality of protrusion portions 12 are disposed between the pair of attachment portions.

Therefore, it is possible to attach the warming patch for foot to the foot sole in a state where the plurality of protrusion portions 12 are in contact with skin of the foot sole.

In other words, the first protrusion portion 12a is disposed on the center portion in the width direction of the first sheet 100a, and both ends in the width direction of the first sheet 100a are non-formed regions in which the protrusion portion 12 (the first protrusion portion 12a) is not formed.

Further, the second protrusion portion 12b is disposed on the center portion in the width direction of the second sheet 100b, and both ends in the width direction of the second sheet 100b are non-formed regions in which the protrusion portion 12 (the second protrusion portion 12b) is not formed.

In addition, the first sheet 100a and the second sheet 100b has a slit 15 at a position near the attachment portion (the adhesive layer 61), respectively. The slit 15 is formed in the base material sheet (the first sheet 10). More specifically, the first sheet 100a and the second sheet 100b have a pair of slits 15 respectively and each slit 15 is disposed near the respective attachment portion.

By forming the slit 15 in the base material sheet, it is possible to easily bend the base material sheet near the attachment portion. Therefore, it is more easily to attach the attachment portion to skin.

In the present embodiment, the formation region of the adhesive layer 61 and the arrangement region of the second sheet 20 are separate, and the slit 15 is formed in the base portion 11 of the first sheet 10 between the formation region of the adhesive layer 61 and the arrangement region of the second sheet 20. The slit 15 is formed to be through the front surface and the back surface of the base 11. The slit 15 may also be in a shape with the width (or may also be an opening), or may actually be in a shape without width. In the case of the present embodiment, the slit 15 is actually without width.

More specifically, the slit 15 is designed to be in a U-shape or V-shape protruding toward the center in the width direction of the warming patch for foot (for example, the respective width direction of the first sheet 100a and the second sheet 100b).

Thus, in the base material sheet (the first sheet 10), in the portion where the slit 15 is formed, it is possible that the closer to the attachment portion (the adhesive layer 61), the less the bending stiffness of the portion is, and the easier bending will be. With this configuration, the arrangement region of the plurality of protrusion portions 12 of the warming patch for foot can be maintained in a plane shape, while the base material sheet can be bent and the attachment portion can be attached to skin. Therefore, it is more easily to attach the attachment portion to skin.

In the case of the present embodiment, for example, as shown in Fig. 12, the user stands up or walk in a state in which the first sheet 100a and the second sheet 100b are attached to the left and the right foot soles respectively, and thus the Yongquan acupuncture point and the insomnia acupuncture point on the left and right foot soles can be pressed by the protrusion portion 12.

In the case of the present embodiment, when the distance from the middle portion corresponding to the middle of the second toe root and the third root of the foot sole to the edge of the heel is set to be L1, it is preferable to respectively press by the plurality of protrusion portions 12: skin in a first circular region which is centered on the width center of the foot sole at L1/3 from the above-mentioned middle portion toward the heel and has a radius of 3 cm, and skin in a second circular region which is centered on the width center of the foot sole at 4×L1/5 from the above-mentioned middle portion toward the heel and has a radius of 3 cm.

More specifically, skin in the first circular region is pressed preferably by the first protrusion portion 12a of the first sheet 100a, and skin in the second circular region is pressed preferably by the second protrusion portion 12b of the second sheet 100b.

In addition, in the case of the present embodiment, when the length of the foot sole is set to be L2, it is preferable to respectively press by the plurality of protrusion portions 12: skin in a first circular region which is centered on the width center of the foot sole at 4×L2/9 from the tiptoe toward the heel and has a radius of 3 cm, and skin in a second circular region which is centered on the width center of the foot sole at 5×L2/6 from the tiptoe toward the heel and has a radius of 3 cm.

More specifically, skin in the first circular region is pressed preferably by the first protrusion portion 12a of the first sheet 100a, and skin in the second circular region is pressed preferably by the second protrusion portion 12b of the second sheet 100b.

In addition, in the case of the present embodiment, when the distance from the middle portion corresponding to the middle of the second toe root and the third toe root of the foot sole to the edge of the heel is set to be LI, and the width of the foot sole at L1/3 from the above-mentioned middle portion toward the heel is set to be W1, and the width of the foot sole at 4×L1/5 from the above-mentioned middle portion toward the heel is set to be W2, it is also preferable to respectively press the plurality of protrusion portions 12: skin in a third circular region which is centered on the width center of the foot sole at L1/3 from the above-mentioned middle portion toward the heel and has an area S1 of (W1)²×π/2, and skin in a fourth circular region which is centered on the width center of the foot sole at 4×L1/5 from the above-mentioned middle portion toward the heel and has an area S2 of (W2)²×π/2.

More specifically, skin in the third circular region is pressed preferably by the first protrusion portion 12a of the first sheet 100a, and skin in the fourth circular region is pressed preferably by the second protrusion portion 12b of the second sheet 100b.

Therefore, it is facilitated to intensively press the periphery of the Yongquan acupuncture point and the periphery of the insomnia acupuncture point.

Further, in the case of the present embodiment, when the length of the foot sole is set to be L2, the width of the foot sole at 4 × L2/9 from the tiptoe to the heel is set to be W1, and the width of the foot sole at 5×L2/6 from the tiptoe to the heel is set to be W2, it is also preferable to respectively press through the plurality of protrusion portions 12: skin in a third circular region which is centered on the width center of the above-mentioned foot sole at 4×L2/9 from the tiptoe toward the heel and has an area S3 of (W1)²×π/2, and skin in a fourth circular region which is centered on the width center of the foot sole at 5×L2/6 from the tiptoe toward the heel and has an area S4 of (W2)²×π/2.

More specifically, skin in the third circular region is pressed preferably by the first protrusion portion 12a of the first sheet 100a, and skin in the fourth circular region is pressed preferably by the second protrusion portion 12b of the second sheet 100b.

Therefore, it is facilitated to intensively press the periphery of the Yongquan acupuncture point and the periphery of the insomnia acupuncture point.

### (Fourth embodiment)

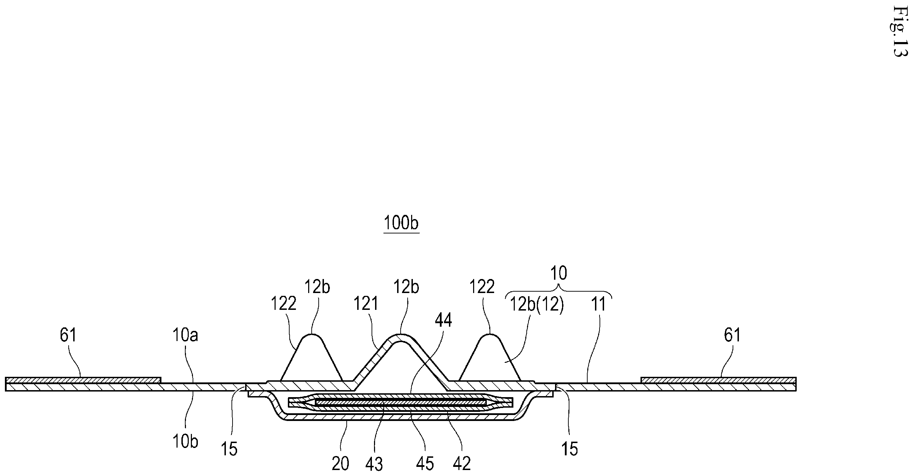

Next, a fourth embodiment will be described with reference to Fig. 13.

In the case of the present embodiment, the warming patch for foot is provided with the warming portion 42 held between the first sheet 10 and the second sheet 20. Although Fig. 13 shows an example in which the warming patch for foot is the second sheet 100b, the warming patch for foot may either be the second sheet 100b or be a sheet in a footprint shape as described in the first embodiment.

Since the warming patch for foot of the present embodiment includes the nonwoven fabric, the user can stretch it a little by tensing and the like. Thus, the user can finely adjust the position of the protrusion portion 12 to fit the acupuncture point of the user.

In the case of the present embodiment, the first sheet 100a and the second sheet are used separately, so that the position can be adjusted according to the size of the foot sole of the user. That is, the protrusion for foot is usually arranged on a sheet having the same size as the foot sole, so that the protrusions for foot in different sizes have to be prepared according to the size of the foot, which is very inconvenient. However, the warming patch for foot of the present embodiment is applicable to any foot size, and is economical and practical.

The warming portion 42 is provided with, for example: a first cover sheet 44, a second cover sheet 45, and a sheet-shaped heat-generating portion 43 held between the first cover sheet 44 and the second cover sheet 45. The first cover sheet 44 and the second cover sheet 45 are overlapped with each other. The first cover sheet 44 and the second cover sheet 45 are joined to each other at, for example, their peripheral sections. Thus, a container receiving the heat-generating portion 43 inside is formed through the first cover sheet 44 and the second cover sheet 45. The first cover sheet 44 and the second cover sheet 45 may be joined by adhesion or bonding, or may be joined by heat sealing.

Of the first cover sheet 44 and the second cover sheet 45, the first cover sheet 44 is disposed on the side of the first sheet 10, that is, on skin side of the foot sole in the state where the warming patch for foot is disposed below the foot sole, and the second cover sheet 45 is disposed on the side of the second sheet 20, that is, on the opposite side of skin side of the user in the state where the warming patch for foot is disposed below the foot sole.

In addition, the invention is not limited to this example, and the warming portion 42 may not have the first cover sheet 44 and the second cover sheet 45. In this case, the container receiving the heat-generating portion 43 inside is formed through, for example, the first sheet 10 and the second sheet 20. In this case, the first sheet 10 has the function of the first cover sheet 44 (the air permeability of the first cover sheet 44, etc.), and the second sheet 20 has the function of the second cover sheet 45 (the air permeability of the second cover sheet 45, etc.).

The heat-generating portion 43 includes the above-mentioned heat-generating material 41.

At least a part of the outer surface of the warming portion 42 is joined to the inner surface of the first sheet 10 or the second sheet 20 at a joined portion (not shown).

There is no particular limit to the form of the heat-generating portion 43, and examples may include the following three types: a coated type, a powder-type, and a paper-produced (paper-made) type.

Here, the coated type of the heat-generating portion 43 is configured by the following method: that is, a coatable heat-generating composition (a heat-generating composition containing iron powder, activated carbon, water, and the like) is coated on crepe paper or a paper laminate, and a polymer sheet is laminated thereon. Instead of using a polymer sheet, a water-absorption layer such as an absorbent polymer, paper or nonwoven fabrics of artificial fibers and the like may be used.

The powder-type of the heat-generating portion 43 is configured by the following method: that is, a powder mixed with iron, activated carbon, water, SAP (Super Absorbent Polymer), inorganic powder and the like is pressed into a sheet shape and sealed between the first cover sheet 44 and the second cover sheet 45.

The paper-produced type of the heat-generating portion 43 is configured by the following method: that is, a heat-generating material constituted by iron powder, activated carbon, and pulp is impregnated with brine, and sealed between the first cover sheet 44 and the second cover sheet 45.

Here, at least one of the first cover sheet 44 and the second cover sheet 45 is constituted by a material having air permeability. In the present embodiment, the air permeability of the first cover sheet 44 is higher than that of the second cover sheet 45. In addition, the second cover sheet 45 may have air permeability or may not actually have air permeability.

In addition, the first cover sheet 44 is a moisture-permeable sheet. On the other hand, the second cover sheet 45 is a moisture-permeable sheet or is a non-moisture-permeable sheet. In the case that the second cover sheet 45 is a moisture-permeable sheet, the air permeability of the second cover sheet 45 may be same as that of the first cover sheet 44, or may be lower than that of the first cover sheet 44, or may be higher than that of the first cover sheet 44.

In addition, the air permeability of the first sheet 10 is preferably higher than that of the first cover sheet 44, and the air permeability of the second sheet 20 is preferably higher than that of the second cover sheet 45. In the case that the second sheet 20 is air-impermeable, the second cover sheet 45 may also be air-impermeable, or the second cover sheet 45 may be air-impermeable.

In addition, in the case that the air permeability of the second cover sheet 45 is lower than that of the first cover sheet 44, the vapor is more easily released to skin.

In the case of the present embodiment, the cavity 13 of the protrusion portion 12 of the first sheet 10 is not filled with the heat-generating material 41, and the inside of the protrusion portion 12 is hollow.

In the present embodiment, the first sheet 10 also has air permeability. More specially, the first sheet 10 also has air permeability at the protrusion portion 12. Therefore, the oxygen can be supplied to the heat-generating material 41 of the heat-generating portion 43 of the warming portion 42 via the first sheet 10 and the first cover sheet 44.

When the warming patch for foot is taken out of the packaging material for use, the heat-generating material 41 of the heat-generating portion 43 of the warming portion 42 is brought into contact with oxygen in the air, the heat-generating portion 43 generates water vapor (the vapor is warm) while it generates heat, and the water vapor is released to the outside via the first cover sheet 44 and the first sheet 10.

Therefore, as in the present embodiment, even if the cavity 13 of the protrusion portion 12 is not filled with the heat-generating material 41, the heat of the warming portion 42 can be rapidly transferred to skin of the living being by the latent heat of the water vapor.

In particular, since the protrusion portion 12 also has air permeability, heat can be transferred to skin by the water vapor released from the protrusion portion 12, and therefore skin can be warmed by the protrusion portion 12 and pressed by the protrusion portion 12.

In addition, the warming patch for foot may not include the second sheet 20 and the warming portion 42 may be attached to the other surface 10b of the first sheet 10.

### (Fifth embodiment)

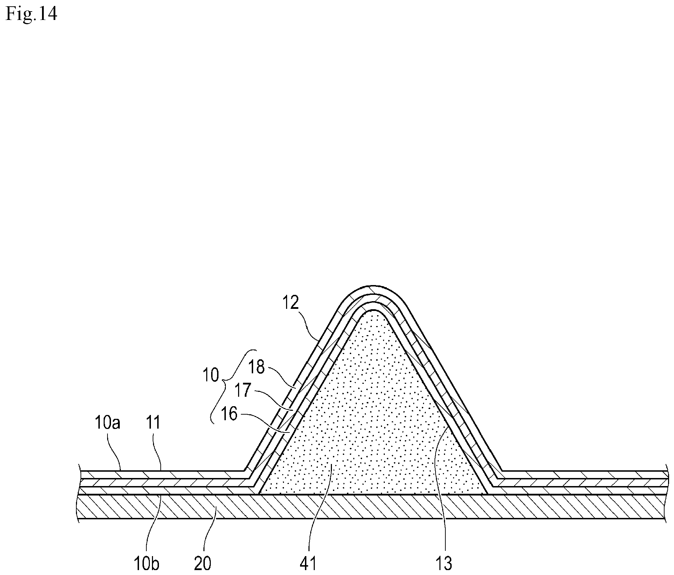

Next, a fifth embodiment will be described with reference to Fig. 14.

Compared with any warming patch for foot of the above-mentioned embodiments, the warming patch for foot of the present embodiment has differences in the first sheet 10 and same configurations in the other aspects.

In each above-mentioned embodiments, examples in which the first sheet 10 is constituted by one nonwoven fabric sheet have been described.

In contrast, in the present embodiment, as illustrated in Fig. 14, the first sheet 10 includes: a nonwoven fabric sheet 16 (a first nonwoven fabric sheet) constituting the outermost layer of the first sheet 10, a nonwoven fabric sheet 18 (a second nonwoven fabric sheet) constituting the other outermost layer of the first sheet 10, and an air-permeable sheet 17 constituting an intermediate layer between the first nonwoven fabric sheet and the second nonwoven fabric sheet.

More specifically, in the case of the present embodiment, the first sheet 10 is in a three-layer structure including the nonwoven fabric sheet 16, the air-permeable sheet 17, and the nonwoven fabric sheet 18.

However, the invention is not limited to this example, and the first sheet 10 may also include layers other than the three layers of the nonwoven fabric sheet 16, the air-permeable sheet 17, and the nonwoven fabric sheet 18. As an example, the first sheet 10 may also be provided with two air-permeable sheets 17 between the nonwoven fabric sheet 16 and the nonwoven fabric sheet 18, and may further be provided with a third nonwoven fabric sheet between the two air-permeable sheets 17, so that the first sheet 10 has a five-layers in total structure.

Same as the nonwoven fabric sheet described in the above-mentioned first embodiment, the nonwoven fabric sheet 16 includes fibers constituted by the first resin material and bonding portions constituted by the second resin material and bonding the fibers to each other. In addition, also same as the nonwoven fabric sheet 16, the nonwoven fabric sheet 18 includes fibers constituted by the first resin material and bonding portions constituted by the second resin material and bonding the fibers to each other.

That is, each of the first nonwoven fabric sheet and the second nonwoven fabric sheet includes: fibers constituted by the first resin material; and a bonding portion constituted by a second resin material and bonding the fibers to each other.

However, the first resin material constituting the nonwoven fabric sheet 16 and the first resin material constituting the nonwoven fabric sheet 18 may be the same material or may be different materials.

In addition, the second resin material constituting the nonwoven fabric sheet 16 and the second resin material constituting the nonwoven fabric sheet 18 may be the same material or may be different materials.

In the present embodiment, for example, the nonwoven fabric sheet 16 and the nonwoven fabric sheet 18 are constituted by the same materials, the first resin material constituting the nonwoven fabric sheet 16 and the first resin material constituting the nonwoven fabric sheet 18 are the same material, and the second resin material constituting the nonwoven fabric sheet 16 and the second resin material constituting the nonwoven fabric sheet 18 are also the same material.

In addition, same as the weight per unit area of the nonwoven fabric sheet 16, the weight per unit area of the nonwoven fabric sheet 18 may be appropriately set.

In addition, in the present embodiment, the fibers constituting the nonwoven fabric sheet 16 may also be in a core-sheath structure including a core constituted by the first resin material and a sheath constituted by the second resin material.

In addition, the fibers constituting the nonwoven fabric sheet 18 may similarly be in a core-sheath structure including a core constituted by the first resin material and a sheath constituted by the second resin material.

The air-permeable sheet 17 is constituted by a third resin material which has a higher melting point than that of the second resin material.

There is no particular limit to the air permeability of the air-permeable sheet 17, and for example, the moisture permeability of the air-permeable sheet 17 is preferably from 100 g/(m²·24h) to 13000 g/(m²·24h), and particularly preferably from 200 g/(m²·24h) to 8000 g/(m²·24h) By setting the moisture permeability of the air-permeable sheet 17 to be in such a range, oxygen gas can be rapidly supplied to the heat-generating material 41 through the first sheet 10 when the warming patch for foot 100 is taken out of the packaging material, heat and water vapor are rapidly generated from the heat-generating material 41, and the duration time of heat generation can be sufficiently prolonged. The moisture permeability of the air-permeable sheet 17 may be measured by, for example, the JIS (Z0208) CaCl₂ method, and the measurement conditions may be set to be 40°C, 90% RH.

The air-permeable sheet 17 may have air permeability over its entire surface or may have air permeability partly.

The weight per unit area of the air-permeable sheet 17 is preferably from 10 g/m² to 200 g/m², and particularly preferably from 20 g/m² to 100 g/m². By setting the weight per unit area of the air-permeable sheet 17 to be in such a range, heat and water vapor can be rapidly generated when the warming patch for foot 100 is taken out of the packaging material, and the duration time of heat generation can be sufficiently prolonged.

Examples of the air-permeable sheet 17 may include: a sheet in which vent holes are mechanically formed in a sheet constituted by a resin of polyolefin such as polyethylene or polypropylene or polyester, polyamide, polyurethane, polystyrene, a copolymer of polyethylene-vinyl acetate, and the like; a sheet in which fine air holes are formed by interfacially peeling of a mixed sheet of these resins and an inorganic filler by extension; a sheet in which fine air holes are formed by interfacially peeling of a crystal structure thereof; a sheet in which fine air holes are communicated by continuous bubbles generated by foam molding; and the like. In addition, examples of the air-permeable sheet 17 may further include: nonwoven fabrics, woven fabrics, synthetic papers, papers, and the like made of synthetic paper pulp such as polyolefin, wood pulp, rayon, semi-synthetic fibers such as acetate fibers, Vinylon fibers, polyester fibers, and the like. A plurality of air-permeable sheets 17 may also be overlapped for use.

More specifically, a sheet in which polypropylene and calcium carbonate are mixed is interfacially peeled by extension, thereby forming fine air pores in the mixed sheet, is preferable to use as the air-permeable sheet 17.

In the present embodiment, the following description will be given to the air-permeable sheet 17 constituted by extending a mixed sheet of polypropylene and calcium carbonate.

Next, the method for producing the warming patch for foot of this embodiment will be briefly explained.

The method for producing the warming patch for foot of the present embodiment includes: the process of forming the protrusion portion 12 on the first sheet 10 by pressing, and a process of filling the cavities 13 of the protrusion portion 12 with the heat-generating material 41.

In order to form the protrusion portion 12 on the first sheet 10, firstly, a nonwoven fabric sheet as the material of the nonwoven fabric sheet 16, the air-permeable sheet 17 and a nonwoven fabric sheet as the material of the nonwoven fabric sheet 18 are prepared, and then the three sheets are laminated in a manner that the air-permeable sheet 17 is located between the two nonwoven fabric sheets.

As described above, the nonwoven fabric sheet includes, for example, the first fibers constituted by the first resin material and the second fibers constituted by the second resin material. However, the fibers constituting the nonwoven fabric sheet may also be in a core-sheath structure including a core constituted by the first resin material and a sheath constituted by the second resin material.

Then, the first sheet 10 on which the protrusion portion 12 is formed is formed by hot stamping the laminate of the three sheets.

In this embodiment, the temperature of the hot stamping is also set to be a temperature between the melting point of the first resin material and the melting point of the second resin material. That is, the temperature of the hot stamping is set to be a temperature lower than the melting point of the first resin material and higher than the melting point of the second resin material.

Thus, the second resin material is melted while the first resin material is not melted, so the fibers constituted by the first resin material (the fibers may also be core portions of the core-sheath structure) are bonded to each other by the melted second resin material. That is, a bonding portion is formed, in which the fibers constituted by the first resin material are bonded to each other by the melted second resin material.

Therefore, not only the air permeability of the nonwoven fabric sheets 16 and 18, but also the stiffness of the nonwoven fabric sheets 16 and 18 can be sufficiently ensured, so that the air permeability and the stiffness of the first sheet 10 can be sufficiently ensured. That is, the air permeability and the stiffness of the base portion 11 can be sufficiently ensured, and for the protrusion portion 12, the air permeability from the base end to the top end of the protrusion portion 12 can also be ensured, and the entire protrusion portion 12 can be set to have sufficient stiffness.

In the case of the present embodiment, it is preferable to set the temperature of the hot stamping to be lower than the melting point of the third resin material included in the air-permeable sheet 17 and lower than the extension temperature of the air-permeable sheet 17. Thus, even after the hot stamping, the air holes of the air-permeable sheet 17 can be maintained, and the air permeability of the air-permeable sheet 17 can be ensured.

In the first sheet 10 after the hot stamping, the second resin material of the nonwoven fabric sheet may or may not be bonded to the air-permeable sheet 17.

In the case of the present embodiment, the second resin material of the nonwoven fabric sheet is not bonded to the air-permeable sheet 17, so that the air permeability of the air-permeable sheet 17 can be well maintained.

In the present embodiment, an example is mainly described in which a sheet formed by extending a mixed sheet of polypropylene and calcium carbonate is used as the air-permeable sheet 17, but the invention is not limited to this example. For example, the air-permeable sheet 17 may also be a sheet made by forming a plurality of pores in a resin sheet constituted by the third resin material. That is, the air-permeable sheet 17 is, for example, a resin sheet constituted by the third resin material, and has a plurality of pores through the front and back surfaces of the air-permeable sheet 17.

In addition, at the time of the above-mentioned hot stamping, it is preferable that the nonwoven fabric is bonded to the air-permeable sheet 17 in advance to reinforce the air-permeable sheet 17 before overlapping the nonwoven fabric sheets on both surfaces of the air-permeable sheet 17.

### (Sixth embodiment)

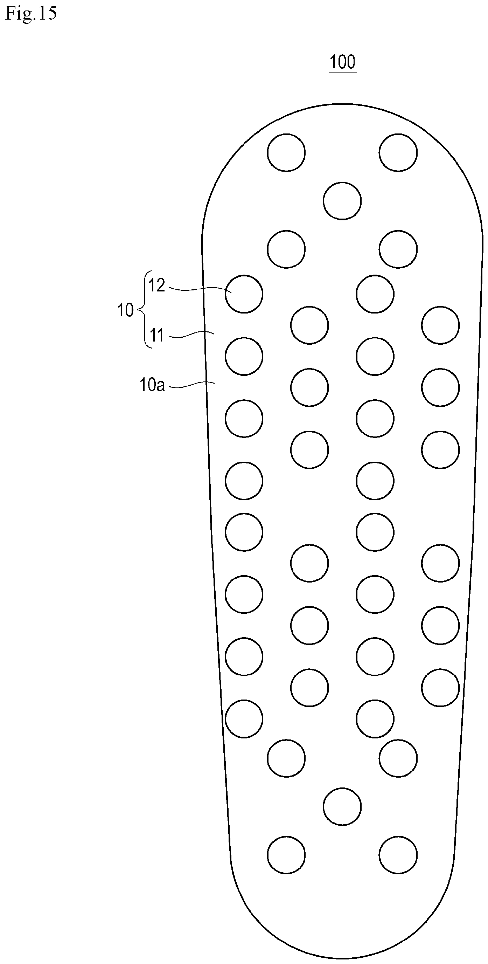

Next, a sixth embodiment will be described with reference to Fig. 15.

In the present embodiment, the protrusion portions 12 are distributed over almost the entire surface of the warming patch for foot 100.

Therefore, the protrusion portion 12 is formed even in a region which does not belong to either "a circular region which is centered on the width center P23 at L1/3 from the middle portion P21 toward the heel and has an area of (W1)²×π/2, or a circular region which is centered on the width center P24 at 4×L1/5 from the middle portion P21 toward the heel and has an area of (W2)²×π/2".

In addition, the protrusion portion 12 is formed even in a region which does not belong to either "a circular region which is centered on the width center P31 at 4×L2/9 from the tiptoe toward the heel of the warming patch for foot 100 and has an area of (W1)²×π/2, or a circular region which is centered on the width center P32 at 5×L2/6 from the tiptoe toward the heel of the warming patch for foot 100 and has an area of (W2)²×π/2".

The other configurations of the warming patch for foot 100 according to the present embodiment is same as those of the warming patch for foot 100 according to the above-mentioned first embodiment or the second embodiment.

The invention is not limited to the above-mentioned embodiments and modifications, but also includes various modifications and improvements that can achieve the object of the invention.

For example, the heat-generating material 41 may be constituted by including an oxidizable metal, a water retaining agent, water, or an absorbent polymer.

When the heat-generating material 41 is constituted by including an absorbent polymer, the remaining water in the heat-generating material 41 can be absorbed by the absorbent polymer. Therefore, when the warming patch for foot 100 is taken out of the packaging material, the heat-generating material 41 can generate heat rapidly.

In the case that the heat-generating material 41 is constituted by including an absorbent polymer, the warming patch for foot 100 may not be provided with the above-mentioned water absorption sheets (the first water absorption sheet 51 and the second water absorption sheet 52) (Fig. 2).

The content of the absorbent polymer in the heat-generating material 41 is preferably from 1% by mass to 12% by mass, more preferably from 2% by mass to 8% by mass. By setting the content of the absorbent polymer in the heat-generating material 41 to be 1% by mass or more, water can be sufficiently absorbed by the absorbent polymer. Further, by setting the content of the absorbent polymer in the heat-generating material 41 to be 12 mass % or less, the content of the oxidizable metal contributing to heat generation in the heat-generating material 41 can be sufficiently ensured.

In addition, in each of the above-mentioned embodiments, an example has been described in which the nonwoven fabric sheet constituting the first sheet 10 includes fibers constituted by the first resin material and bonding portions constituted by the second resin material, which has a lower melting point than the first resin material, and bonding the fibers to each other.

However, the invention is not limited to this example, and the nonwoven fabric sheet constituting the first sheet 10 may be a sheet constituted by fibers of amorphous PET.

Here, the fibers constituting the nonwoven fabric sheet may also include fibers constituted by a material other than amorphous PET. In addition, the fibers of the amorphous PET may contain materials other than the amorphous PET.

The above-mentioned embodiments include the following technical concepts.
<1> A warming patch for foot having a protrusion portion and a warming portion, wherein:
   there are:
   a plurality of the protrusion portions; and
   a base material sheet provided with the plurality of the protrusion portions,
   the base material sheet is constituted by a material including nonwoven fabrics,
   the warming portion has a heat-generating material,
   the protrusion portion is constituted by a material including nonwoven fabrics and is disposed on a surface of the warming patch for foot.
<2> The warming patch for foot as described in <1>, wherein:
   the plurality of the protrusion portions includes:
   a first protrusion portion disposed in the warming patch for foot at a position corresponding to the Yongquan acupuncture point on a sole of a human foot; and
   a second protrusion portion disposed in the warming patch for foot at a position corresponding to the insomnia acupuncture point on the foot sole.
<3> The warming patch for foot as described in <2>, wherein
   there is a plurality of the second protrusion portions,
   the plurality of the second protrusion portions includes: a central protrusion portion disposed at the center of the plurality of the second protrusion portions, and a plurality of peripheral protrusion portions disposed on a circumference with the central protrusion portion as a center.
<4> The warming patch for foot as described in <3>, wherein:
   the plane area of the central protrusion portion of the second protrusion portion is larger than that of the peripheral protrusion portion of the second protrusion portion.
<5> The warming patch for foot as described in <3> or <4>, wherein:
   the height of the central protrusion portion of the second protrusion portion is higher than that of the peripheral protrusion portion of the second protrusion portion.
<6> The warming patch for foot as described in any one of <3> to <5>, wherein:
   the height of the first protrusion portion is higher than the height of either the central protrusion portion of the second protrusion portion or the peripheral protrusion portion of the second protrusion portion.
<7> The warming patch for foot as described in any one of <2> to <6>, wherein:
   there are a plurality of the first protrusion portions and a plurality of the second protrusion portions,
   a configuration form of the plurality of the first protrusion portions and a configuration form of the plurality of the second protrusion portions are different from each other.
<8> The warming patch for foot as described in any one of <2> to <7>, wherein:
   there are a plurality of the first protrusion portions and a plurality of the second protrusion portions,
   the distance between vertexes of the first protrusion portions is greater than the distance between vertexes of the second protrusion portions.
<9> The warming patch for foot as described in any one of <2> to <8>, wherein:
   a top end of the second protrusion portion is designed to be sharper than a top end of the first protrusion portion.
<10> The warming patch for foot as described in any one of <1> to <9>, wherein:
   the protrusion portion is curved into a convex shape on a surface of the warming patch for foot and is formed as a cavity on the other surface.
<11> The warming patch for foot as described in <10>, wherein:
   the heat-generating material is filled in the cavity of the protrusion portion.
<12> The warming patch for foot as described in any one of <1> to <11>, wherein:
   the warming patch for foot is designed to be in a shape of a human footprint.
<13> The warming patch for foot as described in any one of <1> to <12>, wherein:
   when the distance from a middle portion corresponding to a middle of a second toe root and a third toe root to an edge of a heel in the warming patch for foot is set to be L1,
   the plurality of the protrusion portions includes:
      a first protrusion portion disposed in a first circular region which is centered on the width center at L1/3 from the middle portion toward the heel and has a radius of 3 cm; and
      a second protrusion portion disposed in a second circular region which is centered on the width center at 4×L1/5 from the middle portion toward the heel and has a radius of 3 cm.
<14> The warming patch for foot as described in any one of <1> to <12>, wherein:
   when the length of the warming patch for foot is set to L2,
   the plurality of the protrusion portions includes:
      a first protrusion portion disposed in a first circular region which is centered on the width center at 4×L2/9 from the tiptoe toward the heel of the warming patch for foot and has a radius of 3 cm; and
      a second protrusion portion disposed in a second circular region which is centered on the width center at 5×L2/6 from the tiptoe toward the heel of the warming patch for foot and has a radius of 3 cm.
<15> The warming patch for foot as described in any one of <1> to <14>, wherein:
   when the distance from a middle portion corresponding to a middle of a second toe root and a third toe root to an edge of a heel in the warming patch for foot is set to be L1, and the width of the warming patch for foot at L1/3 from the middle portion toward the heel is set to be W1, and the width of the warming patch for foot at 4×L1/5 from the middle portion toward the heel is set to be W2,
   the plurality of the protrusion portions includes:
      a first protrusion portion disposed in a third circular region which is centered on the width center at L1/3 from the middle portion toward the heel and has an area S1 of (W1)²×π/2; and
      a second protrusion portion disposed in a fourth circular region which is centered on the width center at 4×L1/5 from the middle portion toward the heel and has an area S2 of (W2)²×π/2.
<16> The warming patch for foot as described in any one of <1> to <14>, wherein:
   when the length of the warming patch for foot is set to be L2, and the width of the warming patch for foot at 4×L2/9 from the tiptoe toward the heel of the warming patch for foot is set to be W1, and the width of the warming patch for foot at 5×L2/6 from the tiptoe toward the heel of the warming patch for foot is set to be W2,
   the plurality of the protrusion portions includes:
      a first protrusion portion disposed in a third circular region which is centered on the width center at 4×L2/9 from the tiptoe toward the heel of the warming patch for foot and has an area S3 of (W1)²×π/2; and
      a second protrusion portion disposed in a fourth circular region which is centered on the width center at 5×L2/6 from the tiptoe toward the heel of the warming patch for foot and has an area S4 of (W2)²×π/2.
<17> The warming patch for foot as described in <15> or <16>, wherein:
   a region that does not belong to either the third circular region or the fourth circular region is a non-formed region in which the protrusion portion is not formed.
<18> The warming patch for foot as described in any one of <1> to <11>, wherein
   there are:
   a first sheet arranged on a front portion of a foot sole for use; and
   a second sheet arranged on a rear portion of the foot sole for use in a state of being separate from the first sheet,
   the base material sheet includes a first base material sheet constituting the first sheet and a second base material sheet constituting the second sheet,
   the plurality of the protrusion portions includes a first protrusion provided in the first sheet and a second protrusion provided in the second sheet.
<19> The warming patch for foot as described in <18>, wherein:
   the first protrusion portion is disposed at a center portion of the first sheet in the width direction, and
   both ends of the first sheet in the width direction are non-formed regions in which the protrusion portion is not formed,
   the second protrusion portion is disposed at a center portion of the second sheet in the width direction,
   both ends of the second sheet in the width direction are non-formed regions in which the protrusion portion is not formed.
<20> The warming patch for foot as described in <19>, wherein:
   the first sheet is respectively provided with a pair of attachment portions at both ends of the first sheet in the width direction, for attaching the first sheet to the foot sole.
   the second sheet is respectively provided with a pair of attachment portions at both ends of the second sheet in the width direction, for attaching the second sheet to the foot sole.
<21> The warming patch for foot as described in <20>, wherein:
   at a position near the attachment portion, there is a slit formed in the base material sheet.
<22> The warming patch for foot as described in <21>, wherein:
   the slit is designed to be in a U-shape or V-shape protruding toward the center in the width direction of the warming patch for foot.
<23> The warming patch for foot as described in any one of <18> to <22>, wherein:
   there is a plurality set of the first sheets and the second sheets.
<24> A method of using a warming patch for foot, wherein:
   the warming patch for foot described in any one of <1> to <23> is used, and skin of the foot sole in a region corresponding to the Yongquan acupuncture point and skin of the foot sole in a region corresponding to the insomnia acupuncture point are pressed by the plurality of the protrusion portions, respectively.
<25> The method of using a warming patch for foot as described in <24>, wherein:
   the pressing with the plurality of the protrusion portions of the warming patch for foot is started 30 minutes to 120 minutes before bedtime, and the pressing is stopped before bedtime.
<26> The method of using a warming patch for foot as described in <24> or <25>, wherein:
   when the distance from a middle portion corresponding to a middle of a second toe root and a third toe root of a foot sole to an edge of a heel is set to be L1,
   skin in a first circular region and skin in a second circular region are pressed by the plurality of the protrusion portions, respectively, wherein the first circular region is centered on the width center of the foot sole at L1/3 from the middle portion toward the heel and has a radius of 3 cm, and the second circular region is centered on the width center of the foot sole at 4×L1/5 from the middle portion toward the heel and has a radius of 3 cm.
<27> The method of using a warming patch for foot as described in any one of <24> to <26>, wherein:
   when the distance from the middle portion corresponding to a middle of a second toe root and a third toe root of a foot sole to an edge of a heel is set to be L1, and the width of the foot sole at L1/3 from the middle portion toward the heel is set to be W1, and the width of the foot sole at 4×L1/5 from the middle portion toward the heel is set to be W2,
   skin in a third circular region and skin in a fourth circular region are pressed by the plurality of the protrusion portions, respectively, wherein the third circular region is centered on the width center of the foot sole at L1/3 from the middle portion toward the heel and has an area S1 of (W1)²×π/2, and the fourth circular region is centered on the width center of the foot sole at 4×L1/5 from the middle portion toward the heel and has an area S2 of (W2)²×π/2.

   The above-mentioned embodiments also include the following technical concepts.
<28> The warming patch for foot as described in any one of <18> to <23>, wherein:
   there is a plurality of the second protrusion portions,
   the plurality of the second protrusion portions includes: a central protrusion portion disposed at the center of the second sheet in the width direction, and a plurality of peripheral protrusion portions disposed on a circumference with the central protrusion portion as a center.
<29> The warming patch for foot as described in any one of <2> to <9>, any one of <18> to <23>, or <28>, wherein
   the height of the first protrusion portion is higher than the height of the second protrusion portion.
<30> The warming patch for foot as described in any one of <3> to <6> or <28>, wherein:
   the height of the first protrusion portion is higher than the height of at least any one of the peripheral protrusion portions.
<31> The warming patch for foot as described in <1>, wherein:
   both ends of the warming patch for foot in the width direction are respectively provided with a pair of attachment portions for attaching the warming patch for foot to the foot sole,
   the plurality of the protrusion portions is disposed between the pair of attachment portions.
<32> The method of using the warming patch for foot as described in <24> or <25>, wherein:
   when the length of the foot sole is set to be L2,
   skin in a first circular region and skin in a second circular region are pressed by the plurality of the protrusion portions, respectively, wherein the first circular region is centered on the width center of the foot sole at 4× L2/9 from the tiptoe toward the heel and has a radius of 3 cm, and the second circular region is centered on the width center of the foot sole at 5×L2/6 from the tiptoe toward the heel and has a radius of 3 cm.
<33> The method of using a warming patch for foot as described in any one of <24> to <27>, wherein:
   when the length of the foot sole is set to be L2, the width of the foot sole at 4×L2/9 from the tiptoe toward the heel is set to be W1, and the width of the foot sole at 5×L2/6 from the tiptoe toward the heel is set to be W2,
   skin in a third circular region and skin in a fourth circular region are pressed by the plurality of the protrusion portions, respectively, wherein the third circular region is centered on the width center of the foot sole at 4×L2/9 from the tiptoe toward the heel and has an area S3 of (W1)²×π/2, and the fourth circular region is centered on the width center of the foot sole at 5×L2/6 from the tiptoe toward the heel and has an area S4 of (W2)²×π/2.
      The above-mentioned embodiments further include the following technical concepts.
<34> The warming patch for foot as described in any one of the preceding items, wherein:
   the warming patch for foot is provided with: a second sheet sandwiching the warming portion in the middle and laminated on the base material sheet, and a third sheet sandwiching the second sheet in the middle and laminated on the base material sheet.
   the second sheet and the third sheet are partially joined to each other, and in a top view, the second sheet and the third sheet are not joined to each other in an arrangement region of the plurality of the first protrusion portions and an arrangement region of the plurality of the second protrusions.
<35> The warming patch for foot as described in <34>, wherein
   the second sheet and the third sheet are joined to each other at peripheral sections.
<36> The warming patch for foot as described in <34> or <35>, wherein:
   the third sheet is constituted by a nonwoven fabric sheet.
<37> The warming patch for foot as described in <36>, wherein
   for the thickness of the third sheet, the thickness in a region in which the third sheet and the second sheet are not joined is thicker than the thickness in a region in which the third sheet and the second sheet are joined.
   The above-mentioned embodiments also include the following technical concepts.
<38> A warming patch for foot having a protrusion portion and a warming portion, wherein
   there are:
   a plurality of the protrusion portions; and
   a base material sheet provided with the plurality of the protrusion portions,
   the base material sheet is constituted by a material including nonwoven fabrics,
   the warming portion has a heat-generating material,
   the protrusion portion is constituted by a material including nonwoven fabrics and is disposed on the surface of the warming patch for foot,
   the plurality of the protrusion portions includes:
      a first protrusion portion disposed in the warming patch for foot at a position corresponding to the Yongquan acupuncture point on the sole of the human foot; and
      a second protrusion portion disposed in the warming patch for foot at a position corresponding to the insomnia acupuncture point on the foot sole,
      when the length of the warming patch for foot is set to L2,
      the plurality of the protrusion portions includes:
         a first protrusion portion disposed in a first circular region which is centered on the width center at 4×L2/9 from the tiptoe toward the heel of the warming patch for foot and has a radius of 3 cm; and
         a second protrusion portion disposed in a second circular region which is centered on the width center at 5×L2/6 from the tiptoe toward the heel of the warming patch for foot and has a radius of 3 cm.
<39> A warming patch for foot having a protrusion portion and a warming portion, wherein
   there are:
   a plurality of the protrusion portions; and
   a base material sheet provided with the plurality of the protrusion portions,
   the base material sheet is constituted by a material including nonwoven fabrics,
   the warming portion has a heat-generating material,
   the protrusion portion is constituted by a material including nonwoven fabrics and is disposed on the surface of the warming patch for foot,
   the plurality of the protrusion portions includes:
      a first protrusion portion disposed in the warming patch for foot at a position corresponding to the Yongquan acupuncture point on the sole of the human foot; and
      a second protrusion portion disposed in the warming patch for foot at a position corresponding to the insomnia acupuncture point on the foot sole,
      when the length of the warming patch for foot is set to be L2, and the width of the warming patch for foot at 4×L2/9 from the tiptoe toward the heel of the warming patch for foot is set to be W1, and the width of the warming patch for foot at 5×L2/6 from the tiptoe toward the heel of the warming patch for foot is set to be W2,
      the plurality of the protrusion portions includes:
         a first protrusion portion disposed in a third circular region which is centered on the width center at 4×L2/9 from the tiptoe toward the heel of the warming patch for foot and has an area S3 of (W1)²×π/2; and
         a second protrusion portion disposed in a fourth circular region which is centered on the width center at 5×L2/6 from the tiptoe toward the heel of the warming patch for foot and has an area S4 of (W2)²×π/2,
         a region that does not belong to either the third circular region or the fourth circular region is a non-formed region in which the protrusion portion is not formed.
<40> A warming patch for foot having a protrusion portion and a warming portion, wherein
   there are:
   a plurality of the protrusion portions; and
   a base material sheet provided with the plurality of the protrusion portions,
   the base material sheet is constituted by a material including nonwoven fabrics,
   the warming portion has a heat-generating material,
   the protrusion portion is constituted by a material including nonwoven fabrics and is disposed on the surface of the warming patch for foot,
   the plurality of the protrusion portions includes:
      a first protrusion portion disposed in the warming patch for foot at a position corresponding to the Yongquan acupuncture point on the sole of the human foot; and
      a second protrusion portion disposed in the warming patch for foot at a position corresponding to the insomnia acupuncture point on the foot sole,
      when the length of the warming patch for foot is set to be L2, and the width of the warming patch for foot at 4×L2/9 from the tiptoe toward the heel of the warming patch for foot is set to be W1, and the width of the warming patch for foot at 5×L2/6 from the tiptoe toward the heel of the warming patch for foot is set to be W2,
      the plurality of the protrusion portions includes:
         a first protrusion portion disposed in a third circular region which is centered on the width center at 4×L2/9 from the tiptoe toward the heel of the warming patch for foot and has an area S3 of (W1)²×π/2; and
         a second protrusion portion disposed in a fourth circular region which is centered on the width center at 5×L2/6 from the tiptoe toward the heel of the warming patch for foot and has an area S4 of (W2)²×π/2,
         there are a plurality of the first protrusion portions and a plurality of the second protrusion portions,
         a configuration form of the plurality of the first protrusion portions and a configuration form of the plurality of the second protrusion portions are different from each other.
<41> A warming patch for foot having a protrusion portion and a warming portion, wherein
   there are:
   a plurality of the protrusion portions; and
   a base material sheet provided with the plurality of the protrusion portions,
   the base material sheet is constituted by a material including nonwoven fabrics,
   the warming portion has a heat-generating material,
   the protrusion portion is constituted by a material including nonwoven fabrics and is disposed on the surface of the warming patch for foot,
   the plurality of the protrusion portions includes:
      a first protrusion portion disposed in the warming patch for foot at a position corresponding to the Yongquan acupuncture point on the sole of the human foot; and
      a second protrusion portion disposed in the warming patch for foot at a position corresponding to the insomnia acupuncture point on the foot sole,
      when the length of the warming patch for foot is set to be L2, and the width of the warming patch for foot at 4×L2/9 from the tiptoe toward the heel of the warming patch for foot is set to be W1, and the width of the warming patch for foot at 5×L2/6 from the tiptoe toward the heel of the warming patch for foot is set to be W2,
      the plurality of the protrusion portions includes:
         a first protrusion portion disposed in a third circular region which is centered on the width center at 4×L2/9 from the tiptoe toward the heel of the warming patch for foot and has an area S3 of (W1)²×π/2; and
         a second protrusion portion disposed in a fourth circular region which is centered on the width center at 5×L2/6 from the tiptoe toward the heel of the warming patch for foot and has an area S4 of (W2)²×π/2,
         the height of the first protrusion portion is higher than the height of the second protrusion portion.
<42> A warming patch for foot having a protrusion portion and a warming portion, wherein
   there are:
   a plurality of the protrusion portions; and
   a base material sheet provided with the plurality of the protrusion portions,
   the base material sheet is constituted by a material including nonwoven fabrics,
   the warming portion has a heat-generating material,
   the protrusion portion is constituted by a material including nonwoven fabrics and is disposed on the surface of the warming patch for foot,
   a first sheet arranged on a front portion of a foot sole for use; and
   a second sheet arranged on a rear portion of the foot sole for use in a state of being separate from the first sheet,
   the base material sheet includes a first base material sheet constituting the first sheet and a second base material sheet constituting the second sheet,
   the plurality of the protrusion portions includes a first protrusion provided in the first sheet and a second protrusion provided in the second sheet,
   the plurality of the protrusion portions includes:
      a first protrusion portion disposed in the warming patch for foot at a position corresponding to the Yongquan acupuncture point on the sole of the human foot; and
      a second protrusion portion disposed in the warming patch for foot at a position corresponding to the insomnia acupuncture point on the foot sole,
      both ends of the warming patch for foot in the width direction are respectively provided with a pair of attachment portions for attaching the warming patch for foot to the foot sole,
      the plurality of the protrusion portions is disposed between the pair of attachment portions,
      at a position near the attachment portion, there is a slit formed in the base material sheet.
<43> A warming patch for foot having a protrusion portion and a warming portion, wherein
   there are:
   a plurality of the protrusion portions; and
   a base material sheet provided with the plurality of the protrusion portions,
   the base material sheet is constituted by a material including nonwoven fabrics,
   the warming portion has a heat-generating material,
   the protrusion portion is constituted by a material including nonwoven fabrics and is disposed on the surface of the warming patch for foot,
   the plurality of the protrusion portions includes:
      a first protrusion portion disposed in the warming patch for foot at a position corresponding to the Yongquan acupuncture point on the sole of the human foot; and
      a second protrusion portion disposed in the warming patch for foot at a position corresponding to the insomnia acupuncture point on the foot sole,
      the protrusion portion is curved into a convex shape on a surface of the warming patch for foot and is formed as a cavity on the other surface.
<44> A warming patch for foot having a protrusion portion and a warming portion, wherein
   there are:
   a plurality of the protrusion portions; and
   a base material sheet provided with the plurality of the protrusion portions,
   the base material sheet is constituted by a material including nonwoven fabrics,
   the warming portion has a heat-generating material,
   the protrusion portion is constituted by a material including nonwoven fabrics and is disposed on the surface of the warming patch for foot,
   the plurality of the protrusion portions includes:
      a first protrusion portion disposed in the warming patch for foot at a position corresponding to the Yongquan acupuncture point on the sole of the human foot; and
      a second protrusion portion disposed in the warming patch for foot at a position corresponding to the insomnia acupuncture point on the foot sole,
      the protrusion portion is curved into a convex shape on a surface of the warming patch for foot and is formed as a cavity on the other surface,
      the heat-generating material is filled in the cavity of the protrusion portion.
<45> A warming patch for foot having a protrusion portion and a warming portion, wherein
   there are:
   a plurality of the protrusion portions; and
   a base material sheet provided with the plurality of the protrusion portions,
   the base material sheet is constituted by a material including nonwoven fabrics,
   the warming portion has a heat-generating material,
   the protrusion portion is constituted by a material including nonwoven fabrics and is disposed on the surface of the warming patch for foot,
   the plurality of the protrusion portions includes:
      a first protrusion portion disposed in the warming patch for foot at a position corresponding to the Yongquan acupuncture point on the sole of the human foot; and
      a second protrusion portion disposed in the warming patch for foot at a position corresponding to the insomnia acupuncture point on the foot sole,
      when the length of the warming patch for foot is set to L2,
      the plurality of the protrusion portions includes:
         a first protrusion portion disposed in a first circular region which is centered on the width center at 4×L2/9 from the tiptoe toward the heel of the warming patch for foot and has a radius of 3 cm; and
         a second protrusion portion disposed in a second circular region which is centered on the width center at 5×L2/6 from the tiptoe toward the heel of the warming patch for foot and has a radius of 3 cm,
         the warming patch for foot is designed to be in a shape of a human footprint.
<46> A warming patch for foot having a protrusion portion and a warming portion, wherein
   there are:
   a plurality of the protrusion portions; and
   a base material sheet provided with the plurality of the protrusion portions,
   the base material sheet is constituted by a material including nonwoven fabrics,
   the warming portion has a heat-generating material,
   the protrusion portion is constituted by a material including nonwoven fabrics and is disposed on the surface of the warming patch for foot,
   the plurality of the protrusion portions includes:
      a first protrusion portion disposed in the warming patch for foot at a position corresponding to the Yongquan acupuncture point on the sole of the human foot; and
      a second protrusion portion disposed in the warming patch for foot at a position corresponding to the insomnia acupuncture point on the foot sole,
      when the length of the warming patch for foot is set to be L2, and the width of the warming patch for foot at 4×L2/9 from the tiptoe toward the heel of the warming patch for foot is set to be W1, and the width of the warming patch for foot at 5×L2/6 from the tiptoe toward the heel of the warming patch for foot is set to be W2,
      the plurality of the protrusion portions includes:
         a first protrusion portion disposed in a third circular region which is centered on the width center at 4×L2/9 from the tiptoe toward the heel of the warming patch for foot and has an area S3 of (W1)²×π/2; and
         a second protrusion portion disposed in a fourth circular region which is centered on the width center at 5×L2/6 from the tiptoe toward the heel of the warming patch for foot and has an area S4 of (W2)²×π/2,
         there is a plurality of the second protrusion portions,
         the plurality of the second protrusion portions includes: a central protrusion portion disposed at the center of the plurality of the second protrusion portions, and a plurality of peripheral protrusion portions disposed on a circumference with the central protrusion portion as a center,
         the plane area of the central protrusion portion is larger than the plane area of the peripheral protrusion portion,
         there are a plurality of the first protrusion portions and a plurality of the second protrusion portions,
         a configuration form of the plurality of the first protrusion portions and a configuration form of the plurality of the second protrusion portions are different from each other,
         the warming patch for foot is designed to be in a shape of a human footprint.
<47> A warming patch for foot having a protrusion portion and a warming portion, wherein
   there are:
   a plurality of the protrusion portions; and
   a base material sheet provided with the plurality of the protrusion portions,
   the base material sheet is constituted by a material including nonwoven fabrics,
   the warming portion has a heat-generating material,
   the protrusion portion is constituted by a material including nonwoven fabrics and is disposed on the surface of the warming patch for foot,
   the plurality of the protrusion portions includes:
      a first protrusion portion disposed in the warming patch for foot at a position corresponding to the Yongquan acupuncture point on the sole of the human foot; and
      a second protrusion portion disposed in the warming patch for foot at a position corresponding to the insomnia acupuncture point on the foot sole,
      when the length of the warming patch for foot is set to be L2, and the width of the warming patch for foot at 4×L2/9 from the tiptoe toward the heel of the warming patch for foot is set to be W1, and the width of the warming patch for foot at 5×L2/6 from the tiptoe toward the heel of the warming patch for foot is set to be W2,
      the plurality of the protrusion portions includes:
         a first protrusion portion disposed in a third circular region which is centered on the width center at 4×L2/9 from the tiptoe toward the heel of the warming patch for foot and has an area S3 of (W1)²×π/2; and
         a second protrusion portion disposed in a fourth circular region which is centered on the width center at 5×L2/6 from the tiptoe toward the heel of the warming patch for foot and has an area S4 of (W2)²×π/2,
         there is a plurality of the second protrusion portions,
         the plurality of the second protrusion portions includes: a central protrusion portion disposed at the center of the plurality of the second protrusion portions, and a plurality of peripheral protrusion portions disposed on a circumference with the central protrusion portion as a center,
         the height of the central protrusion portion is higher than the height of the peripheral protrusion portion,
         the height of the first protrusion portion is higher than the height of either the central protrusion portion disposed at a center of the plurality of the second protrusion portions or the plurality of the peripheral protrusion portions disposed on a circumference with the central protrusion portion as a center,
         the warming patch for foot is designed to be in a shape of a human footprint.
<48> A warming patch for foot having a protrusion portion and a warming portion, wherein
   there are:
   a plurality of the protrusion portions; and
   a base material sheet provided with the plurality of the protrusion portions,
   the base material sheet is constituted by a material including nonwoven fabrics,
   the warming portion has a heat-generating material,
   the protrusion portion is constituted by a material including nonwoven fabrics and is disposed on the surface of the warming patch for foot,
   the plurality of the protrusion portions includes:
      a first protrusion portion disposed in the warming patch for foot at a position corresponding to the Yongquan acupuncture point on the sole of the human foot; and
      a second protrusion portion disposed in the warming patch for foot at a position corresponding to the insomnia acupuncture point on the foot sole,
      when the length of the warming patch for foot is set to L2,
      the plurality of the protrusion portions includes:
         a first protrusion portion disposed in a first circular region which is centered on the width center at 4×L2/9 from the tiptoe toward the heel of the warming patch for foot and has a radius of 3 cm; and
         a second protrusion portion disposed in a second circular region which is centered on the width center at 5×L2/6 from the tiptoe toward the heel of the warming patch for foot and has a radius of 3 cm,
            there are:
            a first sheet arranged on a front portion of a foot sole for use; and
            a second sheet arranged on a rear portion of the foot sole for use in a state of being separate from the first sheet,
            the base material sheet includes a first base material sheet constituting the first sheet and a second base material sheet constituting the second sheet,
            the plurality of the protrusion portions includes a first protrusion provided in the first sheet and a second protrusion provided in the second sheet.
<49> A warming patch for foot having a protrusion portion and a warming portion, wherein
   there are:
   a plurality of the protrusion portions; and
   a base material sheet provided with the plurality of the protrusion portions,
   the base material sheet is constituted by a material including nonwoven fabrics,
   the warming portion has a heat-generating material,
   the protrusion portion is constituted by a material including nonwoven fabrics and is disposed on the surface of the warming patch for foot,
   the plurality of the protrusion portions includes:
      a first protrusion portion disposed in the warming patch for foot at a position corresponding to the Yongquan acupuncture point on the sole of the human foot; and
      a second protrusion portion disposed in the warming patch for foot at a position corresponding to the insomnia acupuncture point on the foot sole,
      when the length of the warming patch for foot is set to be L2, and the width of the warming patch for foot at 4×L2/9 from the tiptoe toward the heel of the warming patch for foot is set to be W1, and the width of the warming patch for foot at 5×L2/6 from the tiptoe toward the heel of the warming patch for foot is set to be W2,
      the plurality of the protrusion portions includes:
         a first protrusion portion disposed in a third circular region which is centered on the width center at 4×L2/9 from the tiptoe toward the heel of the warming patch for foot and has an area S3 of (W1)²×π/2; and
         a second protrusion portion disposed in a fourth circular region which is centered on the width center at 5×L2/6 from the tiptoe toward the heel of the warming patch for foot and has an area S4 of (W2)²×π/2,
         there is a plurality of the second protrusion portions,
         the plurality of the second protrusion portions includes: a central protrusion portion disposed at the center of the second sheet in the width direction, and a plurality of peripheral protrusion portions disposed on a circumference with the central protrusion portion as a center,
         the plane area of the central protrusion portion is larger than the plane area of the peripheral protrusion portion,
         there are a plurality of the first protrusion portions and a plurality of the second protrusion portions,
         a configuration form of the plurality of the first protrusion portions and a configuration form of the plurality of the second protrusion portions are different from each other,
         there are:
            a first sheet arranged on a front portion of a foot sole for use; and
            a second sheet arranged on a rear portion of the foot sole for use in a state of being separate from the first sheet,
            the base material sheet includes a first base material sheet constituting the first sheet and a second base material sheet constituting the second sheet,
            the plurality of the protrusion portions includes a first protrusion provided in the first sheet and a second protrusion provided in the second sheet.
<50> A warming patch for foot having a protrusion portion and a warming portion, wherein
   there are:
   a plurality of the protrusion portions; and
   a base material sheet provided with the plurality of the protrusion portions,
   the base material sheet is constituted by a material including nonwoven fabrics,
   the warming portion has a heat-generating material,
   the protrusion portion is constituted by a material including nonwoven fabrics and is disposed on the surface of the warming patch for foot,
   the plurality of the protrusion portions includes:
      a first protrusion portion disposed in the warming patch for foot at a position corresponding to the Yongquan acupuncture point on the sole of the human foot; and
      a second protrusion portion disposed in the warming patch for foot at a position corresponding to the insomnia acupuncture point on the foot sole,
      when the length of the warming patch for foot is set to be L2, and the width of the warming patch for foot at 4×L2/9 from the tiptoe toward the heel of the warming patch for foot is set to be W1, and the width of the warming patch for foot at 5×L2/6 from the tiptoe toward the heel of the warming patch for foot is set to be W2,
      the plurality of the protrusion portions includes:
         a first protrusion portion disposed in a third circular region which is centered on the width center at 4×L2/9 from the tiptoe toward the heel of the warming patch for foot and has an area S3 of (W1)²×π/2; and
         a second protrusion portion disposed in a fourth circular region which is centered on the width center at 5×L2/6 from the tiptoe toward the heel of the warming patch for foot and has an area S4 of (W2)²×π/2,
         there is a plurality of the second protrusion portions,
         the plurality of the second protrusion portions includes: a central protrusion portion disposed at the center of the second sheet in the width direction, and a plurality of peripheral protrusion portions disposed on a circumference with the central protrusion portion as a center,
         the height of the central protrusion portion is higher than the height of the peripheral protrusion portion,
         the height of the first protrusion portion is higher than the height of either the central protrusion portion disposed at a center of the plurality of the second protrusion portions or the plurality of the peripheral protrusion portions disposed on a circumference with the central protrusion portion as a center,
         there are:
            a first sheet arranged on a front portion of a foot sole for use; and
            a second sheet arranged on a rear portion of the foot sole for use in a state of being separate from the first sheet,
            the base material sheet includes a first base material sheet constituting the first sheet and a second base material sheet constituting the second sheet,
            the plurality of the protrusion portions includes a first protrusion provided in the first sheet and a second protrusion provided in the second sheet.

### [EXAMPLES]

Hereinafter, examples will be described with the following Tables 1 and 2.

In Example 1 ("the protrusion portion and the warming" in Tables 1 and 2), the testees use a warming patch for foot having the same structure as that described in the first embodiment (Fig. 1). That is, in Example 1, the periphery of the Yongquan acupuncture point and the periphery of the insomnia acupuncture point are partially pressed by the protrusion portion, and the warming effect is applied to the testees by the warming portion.

In Example 2 ("the protrusion portion and the warming on the entire surface" in Tables 1 and 2), the testees use a warming patch for foot having the same structure as that described in the sixth embodiment (Fig. 15) and is without a portion for placing toes as in the first embodiment. That is, in Example 2, the pressing effect is uniformly applied to the entire surface of the foot sole actually by the protrusion portion, and the warming effect is applied to the testees by the warming portion.

In Comparative Example 1 (the "control group" in Tables 1 and 2), the testees used a sheet for foot that is different from the warming patch for foot of the first embodiment in not having a protrusion portion and a warming portion. That is, in Comparative Example 1, neither the pressing effect of the protrusion portion nor the warming effect of the warming portion was applied to the testees.

In Comparative Example 2 ("only the protrusion portion" in Tables 1 and 2), the testees used a sheet for foot with protrusion portions that is different from the warming patch for foot of the first embodiment in not having a warming portion. That is, in Comparative Example 2, only the pressing effect of the protrusion portion is applied to the testees among the warming effect of the warming portion and the pressing effect of the protrusion portion.

In Comparative Example 3 ("only the warming" in Tables 1 and 2), the testees used a warming patch for foot which that is different from the warming patch for foot of the first embodiment in not having a protrusion portion. That is, in Comparative Example 3, only the warming effect of the warming portion is applied to the testees among the warming effect of the warming portion and the pressing effect of the protrusion portion.

**[Table 1]**

| | foot | right foot | | | left foot | | | average | relative degree of recovery |
|---|---|---|---|---|---|---|---|---|---|
| | testee | A | B | C | A | B | C | | |
| Control group | after standing | 75.17 | 131.67 | 85.31 | 80.07 | 97.04 | 88.24 | 92.92 | 100.00 |
| | after recovery | 89.86 | 140.00 | 88.70 | 87.45 | 83.74 | 92.44 | 97.03 | |
| | degree of recovery | 19.54 | 6.33 | 3.97 | 9.22 | -13.71 | 4.76 | 4.43 | |
| the protrusion portion and the warming | after standing | 90.11 | 100.00 | 75.69 | 83.88 | 98.09 | 79.68 | 87.91 | 764.07 |
| | after recovery | 105.49 | 117.53 | 138.67 | 105.13 | 120.38 | 118.73 | 117.66 | |
| | degree of recovery | 17.07 | 17.53 | 83.21 | 25.33 | 22.72 | 49.01 | 33.84 | |
| the protrusion portion and the warming on the entire surface | after standing | 109.87 | 108.47 | 70.89 | 92.51 | 116.87 | 73.90 | 95.42 | 502.55 |
| | after recovery | 106.87 | 122.22 | 115.49 | 117.18 | 125.30 | 112.87 | 116.66 | |
| | degree of recovery | 2.73 | 12.68 | 62.91 | 26.67 | 7.21 | 52.73 | 22.26 | |
| Only the protrusion portion | after standing | 78.57 | 104.02 | 72.46 | 85.96 | 107.69 | 88.59 | 89.55 | 132.47 |
| | after recovery | 90.82 | 101.72 | 79.11 | 89.04 | 107.14 | 100.38 | 94.80 | |
| | degree of recovery | 15.59 | -2.21 | 10.01 | 3.58 | -0.51 | 13.31 | 5.87 | |
| only the warming | after standing | 84.15 | 97.33 | 72.20 | 74.29 | 101.22 | 88.68 | 86.31 | 375.76 |
| | after recovery | 91.55 | 117.33 | 88.29 | 81.07 | 115.24 | 110.57 | 100.68 | |
| | degree of recovery | 8.79 | 20.55 | 22.29 | 9.13 | 13.85 | 24.68 | 16.64 | |

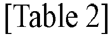

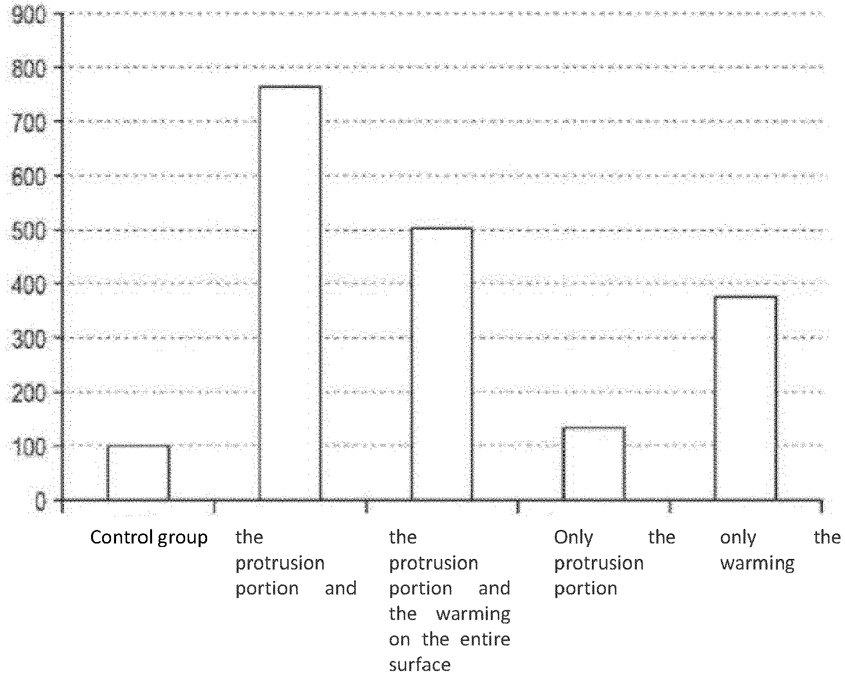

In each of Examples and Comparative Examples, the following evaluation tests were performed on three testees (three testees A, B, and C shown in Table 1).

In the evaluation test, as described below, each testee was subjected to a quiet process, a fatigue process, and a recovery process in this order and then the degree of recovery from fatigue was evaluated.

First, during the quiet process, each testee was quiet in a supine position for 15 minutes. At the end of the quiet process, each testee sat on a chair and a toe holding force was measured. The measured value at this time was set to be the initial value of the toe holding force.

Next, during the fatigue process, each testee maintained a standing posture for 30 minutes.

At the end of the fatigue process, each testee sat on a chair and a toe holding force was measured. The values in the row "after standing" shown in Table 1 represent relative values of the measured values after the end of the fatigue process with respect to the above-mentioned initial values in each of Examples and Comparative Examples (unit: %). Each measurement was performed for the right foot and the left foot, respectively.

The toe holding force was measured by a toe thew measuring device II manufactured by Takei Scientific Instrument Co., Ltd. In a state of sitting on a chair, the foot was placed on the toe thew measuring device II, the heel fixing belt was installed, and the toe traction force was measured in a state of bending the knee join at 90 degrees. Each foot was measured twice, and the higher measured value was employed.

During the recovery process, first, in a state in which the two sheets in each of the Examples and the Comparative Examples (the warming patch for foot, the sheet for foot, or the sheet for foot with protrusion portions) were placed on the floor in a manner of corresponding to the left and the right foot, the testees placed feet on the two sheets respectively. In this state, the testees alternately performed the standing posture and the posture of sitting on the chair every three minutes, and the alternation was repeated, and the repeat was continued for 15 minutes in total. That is, in the recovery process, the posture in the first three minutes was the standing posture, in the next three minutes was the sitting posture, in the following three minutes was the standing posture, in the next following three minutes was the sitting posture, and in the last three minutes was the standing posture.

In addition, when sitting on the chair, the left and the right foot soles of the testees were maintained in a state of being respectively placed on the two sheets of each of Examples and Comparative Examples.

Moreover, at the end of the recovery process, each testee sat on a chair and a toe holding force was measured. The values in the row "after recovery" shown in Table 1 represent relative values of the measured values after the end of the recovery process with respect to the above-mentioned initial values in each of Examples and Comparative Examples. Here, each measurement was performed for the right foot and the left foot, respectively. The measuring method is as described above.

The values in the "degree of recovery" row shown in Table 1 (unit: %) represent improvement in % of the measured value after recovery with the measured value after standing as a reference. The greater the value of the degree of recovery, the higher the degree of recovery of the toe holding force after the recovery process.

The values in the column "average" shown in Table 1 are the respective average values of "after standing", "after recovery" and "degree of recovery".

The "relative degree of recovery" shown in Table 1 represents the values of the degree (unit: %) of recovery in each of Examples and Comparative Examples when the average value of the degree of recovery in Comparative Example 1 (control group) is taken as a reference (100%).

Table 2 is a graphical representation of the "relative degree of recovery" shown in Table 1.

As shown in Table 2, it can be seen that: the degree of recovery in Comparative Example 2 (only the protrusion portion) is slightly improved as compared with Comparative Example 1, and the degree of recovery in Comparative Example 3 (only the warming) is further improved as compared with Comparative Example 2.

In addition, it can be seen that: the degree of recovery is further improved in Example 2 (the protrusion portion and the warming on the entire surface) as compared with Comparative Example 3.

Moreover, it can be seen that: through Example 1 (the protrusion portion and the warming), that is, by partially pressing the periphery of the Yongquan acupuncture point and the periphery of the insomnia acupuncture point through the protrusion portion and by applying the warming effect through the warming portion, the degree of recovery is significantly improved compared with that of Example 2.

### Reference numerals

- 10: first sheet
- 10a: one surface
- 10b: the other surface
- 11: base portion
- 11a: first portion
- 11b: second portion
- 12: protrusion portion
- 12a: first protrusion portion
- 12b: second protrusion portion
- 121: central protrusion portion
- 122: peripheral protrusion portion
- 13: cavity
- 15: slit
- 16: nonwoven fabric sheet
- 17: air-permeable sheet
- 18: nonwoven fabric sheet
- 20: second sheet
- 30: third sheet
- 30a: first portion
- 30b: second portion
- 41: heat-generating material
- 42: warming portion
- 43: heat-generating portion
- 44: first cover sheet
- 45: second cover sheet
- 51: first water absorption sheet
- 52: second water absorption sheet
- 61: adhesive layer
- 70: shoes
- 71: sole of shoes
- 80: foot
- 91: Yongquan acupuncture point
- 92: insomnia acupuncture point
- 100: warming patch for foot
- 100a: first sheet
- 100b: second sheet

## Claims

1. A warming patch for foot having a protrusion portion and a warming portion, wherein:
there are:
a plurality of the protrusion portions; and
a base material sheet provided with the plurality of the protrusion portions,
the base material sheet is constituted by a material including nonwoven fabrics,
the warming portion has a heat-generating material,
the protrusion portion is constituted by a material including nonwoven fabrics and is disposed on a surface of the warming patch for foot.

2. The warming patch for foot according to claim 1, wherein:
the plurality of the protrusion portions includes:
a first protrusion portion disposed in the warming patch for foot at a position corresponding to the Yongquan acupuncture point on a sole of a human foot; and
a second protrusion portion disposed in the warming patch for foot at a position corresponding to the insomnia acupuncture point on the foot sole.

3. The warming patch for foot according to claim 2, wherein:
there is a plurality of the second protrusion portions,
the plurality of the second protrusion portions includes: a central protrusion portion disposed at the center of the plurality of the second protrusion portions, and a plurality of peripheral protrusion portions disposed on a circumference with the central protrusion portion as a center.

4. The warming patch for foot according to claim 3, wherein:
the plane area of the central protrusion portion of the second protrusion portion is larger than that of the peripheral protrusion portion of the second protrusion portion.

5. The warming patch for foot according to claim 3 or 4, wherein:
the height of the central protrusion portion of the second protrusion portion is higher than that of the peripheral protrusion portion of the second protrusion portion.

6. The warming patch for foot according to any one of claims 3 to 5, wherein:
the height of the first protrusion portion is higher than the height of either the central protrusion portion of the second protrusion portion or the peripheral protrusion portion of the second protrusion portion.

7. The warming patch for foot according to any one of claims 2 to 6, wherein:
there are a plurality of the first protrusion portions and a plurality of the second protrusion portions,
a configuration form of the plurality of the first protrusion portions and a configuration form of the plurality of the second protrusion portions are different from each other.

8. The warming patch for foot according to any one of claims 2 to 7, wherein:
there are a plurality of the first protrusion portions and a plurality of the second protrusion portions,
the distance between vertexes of the first protrusion portions is greater than the distance between vertexes of the second protrusion portions.

9. The warming patch for foot according to any one of claims 2 to 8, wherein:
a top end of the second protrusion portion is designed to be sharper than a top end of the first protrusion portion.

10. The warming patch for foot according to any one of claims 1 to 9, wherein:
the protrusion portion is curved into a convex shape on a surface of the warming patch for foot and is formed as a cavity on the other surface.

11. The warming patch for foot according to claim 10, wherein:
the heat-generating material is filled in the cavity of the protrusion portion.

12. The warming patch for foot according to any one of claims 1 to 11, wherein:
the warming patch for foot is designed to be in a shape of a human footprint.

13. The warming patch for foot according to any one of claims 1 to 12, wherein:
when the distance from a middle portion corresponding to a middle of a second toe root and a third toe root to an edge of a heel in the warming patch for foot is set to be L1,
the plurality of the protrusion portions includes:
a first protrusion portion disposed in a first circular region which is centered on the width center at L1/3 from the middle portion toward the heel and has a radius of 3 cm; and
a second protrusion portion disposed in a second circular region which is centered on the width center at 4×L1/5 from the middle portion toward the heel and has a radius of 3 cm.

14. The warming patch for foot according to any one of claims 1 to 12, wherein:
when the length of the warming patch for foot is set to L2,
the plurality of the protrusion portions includes:
a first protrusion portion disposed in a first circular region which is centered on the width center at 4×L2/9 from the tiptoe toward the heel of the warming patch for foot and has a radius of 3 cm; and
a second protrusion portion disposed in a second circular region which is centered on the width center at 5×L2/6 from the tiptoe toward the heel of the warming patch for foot and has a radius of 3 cm.

15. The warming patch for foot according to any one of claims 1 to 14, wherein:
when the distance from a middle portion corresponding to a middle of a second toe root and a third toe root to an edge of a heel in the warming patch for foot is set to be L1, and the width of the warming patch for foot at L1/3 from the middle portion toward the heel is set to be W1, and the width of the warming patch for foot at 4×L1/5 from the middle portion toward the heel is set to be W2,
the plurality of the protrusion portions includes:
a first protrusion portion disposed in a third circular region which is centered on the width center at L1/3 from the middle portion toward the heel and has an area S1 of (W1)²×π/2; and
a second protrusion portion disposed in a fourth circular region which is centered on the width center at 4×L1/5 from the middle portion toward the heel and has an area S2 of (W2)²×π/2.

16. The warming patch for foot according to any one of claims 1 to 14, wherein:
when the length of the warming patch for foot is set to be L2, and the width of the warming patch for foot at 4×L2/9 from the tiptoe toward the heel of the warming patch for foot is set to be W1, and the width of the warming patch for foot at 5×L2/6 from the tiptoe toward the heel of the warming patch for foot is set to be W2,
the plurality of the protrusion portions includes:
a first protrusion portion disposed in a third circular region which is centered on the width center at 4×L2/9 from the tiptoe toward the heel of the warming patch for foot and has an area S3 of (W1)²×π/2; and
a second protrusion portion disposed in a fourth circular region which is centered on the width center at 5×L2/6 from the tiptoe toward the heel of the warming patch for foot and has an area S4 of (W2)²×π/2.

17. The warming patch for foot according to claim 15 or 16, wherein:
a region that does not belong to either the third circular region or the fourth circular region is a non-formed region in which the protrusion portion is not formed.

18. The warming patch for foot according to any one of claims 1 to 11, wherein:
there are:
a first sheet arranged on a front portion of a foot sole for use; and
a second sheet arranged on a rear portion of the foot sole for use in a state of being separate from the first sheet,
the base material sheet includes a first base material sheet constituting the first sheet and a second base material sheet constituting the second sheet,
the plurality of the protrusion portions includes a first protrusion provided in the first sheet and a second protrusion provided in the second sheet.

19. The warming patch for foot according to claim 18, wherein:
the first protrusion portion is disposed at a center portion of the first sheet in the width direction, and
both ends of the first sheet in the width direction are non-formed regions in which the protrusion portion is not formed,
the second protrusion portion is disposed at a center portion of the second sheet in the width direction,
both ends of the second sheet in the width direction are non-formed regions in which the protrusion portion is not formed,

20. The warming patch for foot according to claim 19, wherein:
the first sheet is respectively provided with a pair of attachment portions at both ends of the first sheet in the width direction, for attaching the first sheet to the foot sole.
the second sheet is respectively provided with a pair of attachment portions at both ends of the second sheet in the width direction, for attaching the second sheet to the foot sole.

21. The warming patch for foot according to claim 20, wherein:
at a position near the attachment portion, there is a slit formed in the base material sheet.

22. The warming patch for foot according to claim 21, wherein:
the slit is designed to be in a U-shape or V-shape protruding toward the center in the width direction of the warming patch for foot.

23. The warming patch for foot according to any one of claims 18 to 22, wherein:
there are a plurality set of the first sheets and the second sheets.

24. A method of using a warming patch for foot, wherein:
the warming patch for foot according to any one of claims 1 to 23 is used, and skin of the foot sole in a region corresponding to the Yongquan acupuncture point and skin of the foot sole in a region corresponding to the insomnia acupuncture point are pressed by the plurality of the protrusion portions, respectively.

25. The method of using a warming patch for foot according to claim 24, wherein:
the pressing with the plurality of the protrusion portions of the warming patch for foot is started 30 minutes to 120 minutes before bedtime, and the pressing is stopped before bedtime.

26. The method of using a warming patch for foot according to claim 24 or 25, wherein:
when the distance from a middle portion corresponding to a middle of a second toe root and a third toe root of a foot sole to an edge of a heel is set to be L1,
skin in a first circular region and skin in a second circular region are pressed by the plurality of the protrusion portions, respectively, wherein the first circular region is centered on the width center of the foot sole at L1/3 from the middle portion toward the heel and has a radius of 3 cm, and the second circular region is centered on the width center of the foot sole at 4×L1/5 from the middle portion toward the heel and has a radius of 3 cm.

27. The method of using a warming patch for foot according to any one of claims 24 to 26, wherein:
when the distance from a middle portion corresponding to a middle of a second toe root and a third toe root of a foot sole to an edge of a heel is set to be L1, and the width of the foot sole at L1/3 from the middle portion toward the heel is set to be W1, and the width of the foot sole at 4×L1/5 from the middle portion toward the heel is set to be W2,
skin in a third circular region and skin in a fourth circular region are pressed by the plurality of the protrusion portions, respectively, wherein the third circular region is centered on the width center of the foot sole at L1/3 from the middle portion toward the heel and has an area S1 of (W1)²×π/2, and the fourth circular region is centered on the width center of the foot sole at 4×L1/5 from the middle portion toward the heel and has an area S2 of (W2)²×π/2.
